# EUROPEAN PATENT SPECIFICATION

(11) **EP 4 482 617 B1**
(45) Date of publication and mention of the grant of the patent: **06.08.2025**
(21) Application number: 23745007.7
(22) Date of filing: 28.06.2023
(51) Int. Cl.: B01J 19/00

(54) **METHOD OF GENERATING ARRAYS USING MICROFLUIDICS AND PHOTOLITHOGRAPHY**
VERFAHREN ZUR ERZEUGUNG VON ARRAYS UNTER VERWENDUNG VON MIKROFLUIDIK UND PHOTOLITHOGRAPHIE
PROCÉDÉ DE GÉNÉRATION DE RÉSEAUX À L'AIDE DE LA MICROFLUIDIQUE ET DE LA PHOTOLITHOGRAPHIE

(30) Priority: 29.06.2022 US 202263356735 P
(43) Date of publication of application: 01.01.2025
(60) Divisional application: 25180565.1
(73) Proprietor: 10X Genomics, Inc., Pleasanton, CA 94588-3260 (US)
(72) Inventor: SHAH, Preyas, Pleasanton, California 94588-3260 (US)
(74) Representative: J A Kemp LLP
(86) International application number: PCT/US2023/069241
(87) International publication number: WO 2024/006814

(56) References cited:
- WO-A1-2021/167807
- WO-A1-2022/103499
- US-A1- 2021 332 351
- US-A1- 2022 023 820

## Description

### FIELD

The present disclosure relates in some aspects to methods for manufacturing molecular arrays using microfluidics and photolithography and related compositions, devices, and systems.

### BACKGROUND

Arrays of nucleic acids are an important tool in the biotechnology industry and related fields. There are two main ways of producing nucleic acid arrays in which the immobilized nucleic acids are covalently attached to a substrate surface, e.g., via *in situ* synthesis in which a nucleic acid polymer is grown on the surface of the substrate in a step-wise, nucleotide-by-nucleotide fashion, or via deposition of a full, pre-synthesized nucleic acid/polypeptide, cDNA fragment, etc., onto the surface of the array.

At present, there remains a need to generate molecular arrays for analyzing at high resolution the spatial expression patterns of large numbers of genes, proteins, or other biologically active molecules simultaneously. Provided are methods, uses and articles of manufacture that meet these needs. WO 2022/103499 discloses a method for enzymatic synthesis of polynucleotides.

### SUMMARY

Nucleic acid arrays in which a plurality of distinct or different nucleic acids are patterned on a solid support surface find use in a variety of applications, including gene expression analysis, drug screening, nucleic acid sequencing, mutation analysis, and the like. A feature of many arrays that have been developed is that each of the distinct nucleic acids of the array is stably attached to a discrete location on the array surface, such that its position remains constant and known throughout the use of the array. Stable attachment is achieved in a number of different ways, including covalent bonding of a nucleic acid polymer to the support surface and non-covalent interaction of the nucleic acid polymer with the surface.

Provided herein are methods for producing a molecular array (e.g., nucleic acid array) using a combination of microfluidics and photolithography, where the nucleic acid polymer is grown *in situ* on the surface of a substrate in a step-wise fashion. Instead of base-by-base synthesis, an immobilized nucleic acid can be synthesized by sequentially attaching oligonucleotides each of at least four nucleotides in length, e.g., through oligonucleotide hybridization and ligation, and microfluidics and photolithography can be used to guide the hybridization and ligation to achieve desired sequence diversity in the array comprising extended immobilized nucleic acid molecules.

In some aspects, provided herein is a method for generating an immobilized nucleic acid, which can include a) delivering a first oligonucleotide of at least four nucleotides in length through a first microfluidic channel to a corresponding first area on a substrate, whereby the first oligonucleotide is attached to an oligonucleotide molecule immobilized in the first area to generate an extended oligonucleotide molecule, optionally wherein the first oligonucleotide comprises a first barcode sequence; b) delivering a second oligonucleotide of at least four nucleotides in length through a second microfluidic channel to a corresponding second area on the substrate, whereby the second oligonucleotide is attached to the extended oligonucleotide molecule to generate a further extended oligonucleotide molecule in an overlapping area between the first and second areas, optionally wherein the second oligonucleotide comprises a second barcode sequence; c) irradiating the substrate to render oligonucleotide molecules in one or more regions on the substrate available for oligonucleotide attachment, whereas oligonucleotide molecules in one or more other regions on the substrate are not available for oligonucleotide attachment; and d) attaching a third oligonucleotide of at least four nucleotides in length to the further extended oligonucleotide molecule to generate an immobilized nucleic acid on the substrate, optionally wherein the third oligonucleotide comprises a third barcode sequence and the immobilized nucleic acid comprises the first, second, and third barcode sequences.

In any of the preceding embodiments, the first oligonucleotide can include a sequence that hybridizes to a first splint which in turn hybridizes to the oligonucleotide molecule. In any of the preceding embodiments, the first oligonucleotide can be ligated to the oligonucleotide molecule using the first splint as a template to generate the extended oligonucleotide molecule. In any of the preceding embodiments, the second oligonucleotide can include a sequence that hybridizes to a second splint which in turn hybridizes to the extended oligonucleotide molecule. In any of the preceding embodiments, the second oligonucleotide can be ligated to the extended oligonucleotide molecule using the second splint as a template to generate the further extended oligonucleotide molecule. In any of the preceding embodiments, the third oligonucleotide can include a sequence that hybridizes to a third splint which in turn hybridizes to the further extended oligonucleotide molecule. In any of the preceding embodiments, the third oligonucleotide can be ligated to the further extended oligonucleotide molecule using the third splint as a template to generate the immobilized nucleic acid comprising the first, second, and third barcode sequences. In any of the preceding embodiments, prior to the irradiating in step c), the oligonucleotide molecules in the one or more regions can be protected from hybridization and/or ligation. In any of the preceding embodiments, during and after the irradiating in step c), the oligonucleotide molecules in the one or more other regions can be protected from hybridization and/or ligation.

In any of the preceding embodiments, the oligonucleotide molecules can be protected from hybridization and/or ligation by a photoresist covering the oligonucleotide molecules. In any of the preceding embodiments, the photoresist in irradiated regions can be removed and the photoresist in masked or non-irradiated regions can be not removed. In any of the preceding embodiments, the oligonucleotide molecules can be protected from hybridization and/or ligation by a protective group of each oligonucleotide molecule. In any of the preceding embodiments, the protective group can be a photo-cleavable protective group. In any of the preceding embodiments, the photo-cleavable protective group in the irradiated regions can be cleaved and the photo-cleavable protective group in masked or non-irradiated regions can be not cleaved.

In any of the preceding embodiments, the oligonucleotide molecules can be protected from hybridization and/or ligation by a polymer binding to the oligonucleotide molecules. In any of the preceding embodiments, the polymer can be a photo-cleavable polymer, optionally wherein the polymer binds to the oligonucleotide molecules in a non-sequence specific manner. In any of the preceding embodiments, the photo-cleavable polymer in the irradiated regions can be cleaved and the photo-cleavable polymer in masked or non-irradiated regions can be not cleaved.

In any of the preceding embodiments, the substrate can be irradiated through a photomask comprising openings that correspond to regions on the substrate, and one or more of the regions are in the overlapping area between the first area and the second area. In any of the preceding embodiments, the substrate can be irradiated in multiple cycles, each cycle for irradiating one or more regions that are different from the region(s) irradiated in another cycle. In any of the preceding embodiments, the overlapping area between the first area and the second area can be irradiated in multiple cycles, each cycle for irradiating one or more regions in the overlapping area that are different from the region(s) irradiated in another cycle. In any of the preceding embodiments, the photomask can be translated from a first position to a second position relative to the substrate, each position for a cycle of irradiating the substrate.

In any of the preceding embodiments, the first and second microfluidic channels form an the angle of about 90 degrees, about 80 degrees, about 70 degrees, about 60 degrees, about 50 degrees, about 40 degrees, about 30 degrees, about 20 degrees, or about 10 degrees. In any of the preceding embodiments, the first and second microfluidic channels can be provided in the same microfluidic device or in separate microfluidic devices. In any of the preceding embodiments, the width of the first and/or second microfluidic channels can be about 5 µm, about 10 µm, about 50 µm, about 100 µm, about 150 µm, about 200 µm, about 250 µm, about 300 µm, about 350 µm, about 400 µm, about 450 µm, or about 500 µm. In any of the preceding embodiments, the depth of the first and/or second microfluidic channels can be about 5 µm, about 10 µm, about 50 µm, about 100 µm, about 150 µm, about 200 µm, about 250 µm, about 300 µm, about 350 µm, about 400 µm, about 450 µm, or about 500 µm.

In any of the preceding embodiments, the immobilized nucleic acid can include, in the 3' to 5' direction or in the 5' to 3' direction: the first barcode sequence, the second barcode sequence, and the third barcode sequence. In any of the preceding embodiments, the immobilized nucleic acid can include, in the 3' to 5' direction or in the 5' to 3' direction: a primer sequence or partial primer sequence, the first barcode sequence, the second barcode sequence, the third barcode sequence, a unique molecular identifier (UMI), and a capture sequence. In any of the preceding embodiments, the primer sequence or partial primer sequence can be used for sequencing the immobilized nucleic acid or a portion thereof, or for sequencing a complement of the immobilized nucleic acid or portion thereof. In any of the preceding embodiments, the capture sequence can include a poly(dT) sequence.

In any of the preceding embodiments, the immobilized nucleic acid can be 3' immobilized on the substrate. In any of the preceding embodiments, the immobilized nucleic acid can be 5' immobilized on the substrate. In any of the preceding embodiments, the immobilized nucleic acid can be not generated in a cell or tissue sample. In any of the preceding embodiments, the substrate can be a chip, a wafer, a die, or a slide and the immobilized nucleic acid can be generated in the absence of a cell or tissue sample on the substrate.

### BRIEF DESCRIPTION OF THE DRAWINGS

The drawings illustrate certain features and advantages of this disclosure. These embodiments are not intended to limit the scope of the appended claims in any manner.

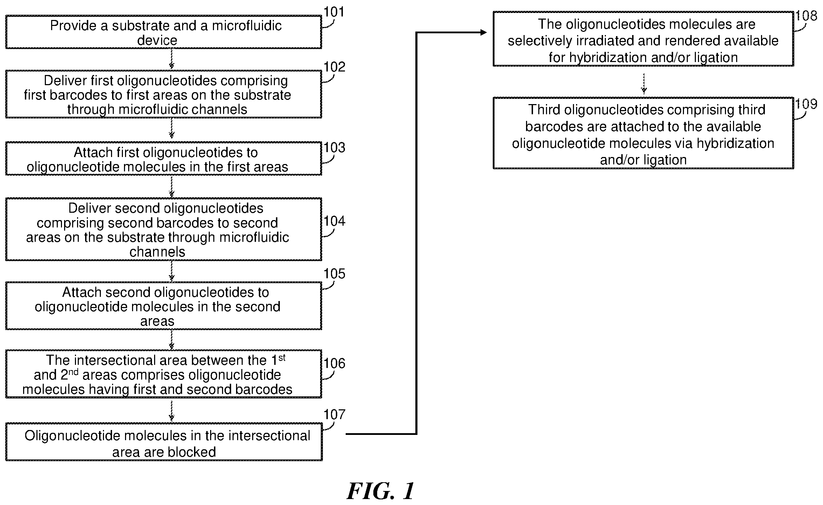

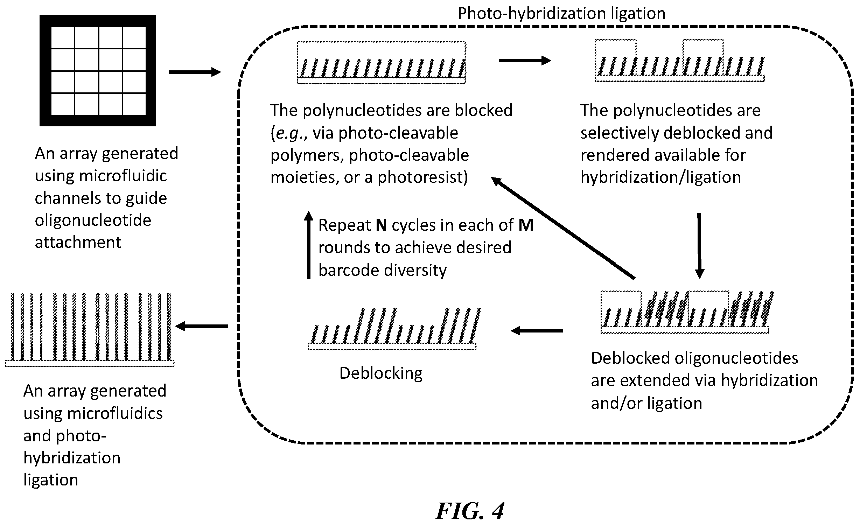

**FIG. 1** is a flow chart showing a representative work flow of making an oligonucleotide array using microfluidic channels and photolithography-based methods, such as the photo-hybridization ligation described in **FIG. 4****.** The work flow progresses through a series of steps described in the boxes labeled 101 through 109. The step in the box labeled 101 is to provide a substrate and a microfluidic device. The step in the box labeled 102 is to deliver first oligonucleotides comprising first barcodes to first areas on the substrate through microfluidic channels. The step in the box labeled 103 is to attach first oligonucleotides to oligonucleotide molecules in the first areas. The step in the box labeled 104 is to deliver second oligonucleotides comprising second barcodes to second areas on the substrate through microfluidic channels. The step in the box labeled 105 is to attach second oligonucleotides to oligonucleotide molecules in the second areas. The step in the box labeled 106 is that the intersectional area between the first and second areas comprises oligonucleotide molecules having first and second barcodes. The step in the box labeled 107 is that the oligonucleotide molecules in the intersectional area are blocked. The step in the box labeled 108 is that the oligonucleotides molecules are selectively irradiated and rendered available for hybridization and/or ligation. The step in the box labeled 109 is that the third oligonucleotides comprising third barcodes are attached to the available oligonucleotides molecules via hybridization and/or ligation.

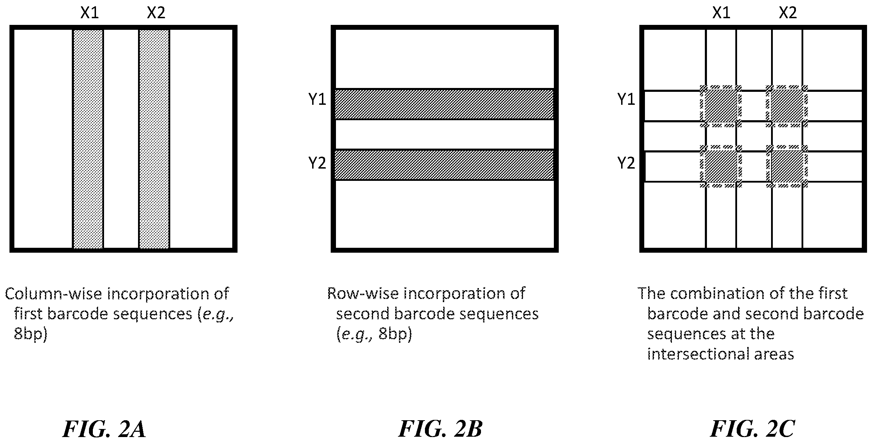

**FIGS. 2A-2C** are graphs showing the incorporation of oligonucleotide sequences using microfluidic channels. **FIG. 2A** shows the incorporation of "part A" barcode sequences using a plurality of first microfluidic channels to oligonucleotide molecules in corresponding first areas (e.g., the shaded areas labeled X1 and X2), shown here as *column-*wise incorporation of first barcode sequences (e.g., 8 base pairs (bp) long). **FIG. 2B** shows the incorporation of "part B" barcode sequences using a plurality of second microfluidic channels to oligonucleotide molecules in corresponding second areas (e.g., the shaded areas labeled Y1 and Y2), shown here as row-wise incorporation of second barcode sequences (e.g., 8 bp). **FIG. 2C** shows the combination of the first barcode sequences shown in **FIG. 2A** and the second barcode sequences shown in **FIG. 2B** at the overlapping areas where the first and second areas intersect. The combination of the first barcode and second barcode sequences at the intersectional areas is shown in the shaded areas.

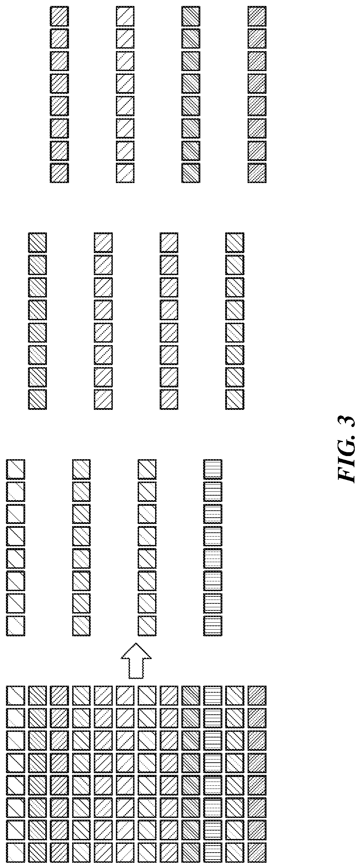

**FIG. 3** is a schematic graph showing the first microfluidic channels are substantially parallel to one another, and a subset of the first microfluidic channels are utilized in each of multiple steps to deliver the first oligonucleotides to the corresponding first areas, in a staggered manner, optionally with washing and drying the microfluidic channels for reuse for the multiple steps. Each patterned box represents a single microfluidic channel. Each row of microfluidic channels with the same pattern represents microfluidic channels having the same oligonucleotides. The arrow represents uses of subsets of microfluidic channels at a time. To the left of the arrow is an exemplary full set of microfluidic channels, and each of the staggered subsets shown to the right of the arrow show individual steps in which staggered subsets of microfluidic channels are used at a time.
**FIG. 4** is a schematic graph showing oligonucleotides on an array generated by microfluidic channels are further extended by photolithography-guided oligonucleotide hybridization and ligation (photo-hybridization ligation). The arrows show an exemplary order of a work flow to generate the array. The top left shows a starting point comprising an array generated using microfluidic channels to guide oligonucleotide attachment. The area on the right outlined in a dashed line shows photo-hybridization ligation. Within that area, the top left portion shows that the polynucleotides are blocked (*e.g.,* via photo-cleavable polymers, photo-cleavable moieties, or a photoresist); the top right area shows that the polynucleotides are selectively deblocked and rendered available for hybridization/ligation; the bottom right portion shows that deblocked oligonucleotides are extended via hybridization and/or ligation; the bottom left section shows deblocking; and the arrow pointing back to the top left portion shows that the cycle may be repeated by, for instance, repeating **N** cycles in each of **M** rounds to achieve desired barcode diversity. Finally the bottom left portion outside the dashed line shows the array generated using microfluidics and photo-hybridization ligation via the displayed work flow. The deblocking method can depend on the blocking method. For instance, the deblocking can comprise: photo-cleaving a polymer that blocks an oligonucleotide in a prior cycle from hybridization and ligation; removing a photo-cleavable moiety of an oligonucleotide that blocks the oligonucleotide in a prior cycle from hybridization and ligation; or removing a photoresist that blocks the oligonucleotide in a prior cycle from hybridization and ligation.

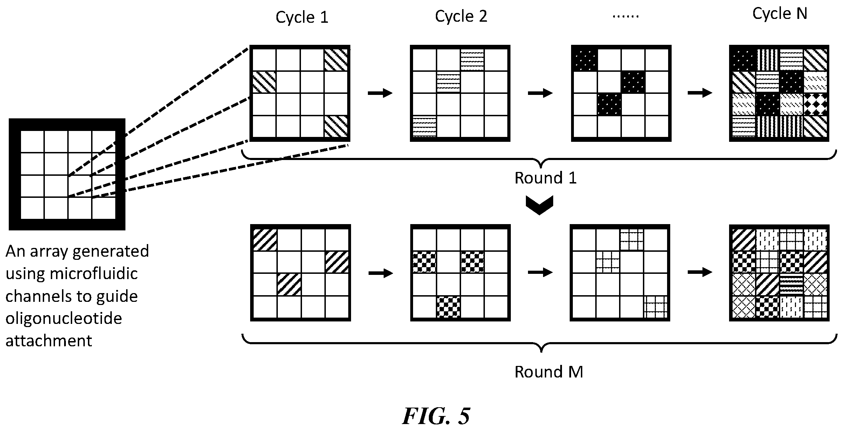

**FIG. 5** is a schematic representation of further patterning an oligonucleotide array generated using microfluidic channels to guide oligonucleotide attachment (left), followed by using a photomask and photolithography to guide oligonucleotide hybridization and ligation in sequential cycles (*e.g.,* Cycle 1, Cycle 2, to Cycle N) in each of M rounds (e.g., Round 1 to Round M), wherein the results of each cycle are shown shaded in different patterns.

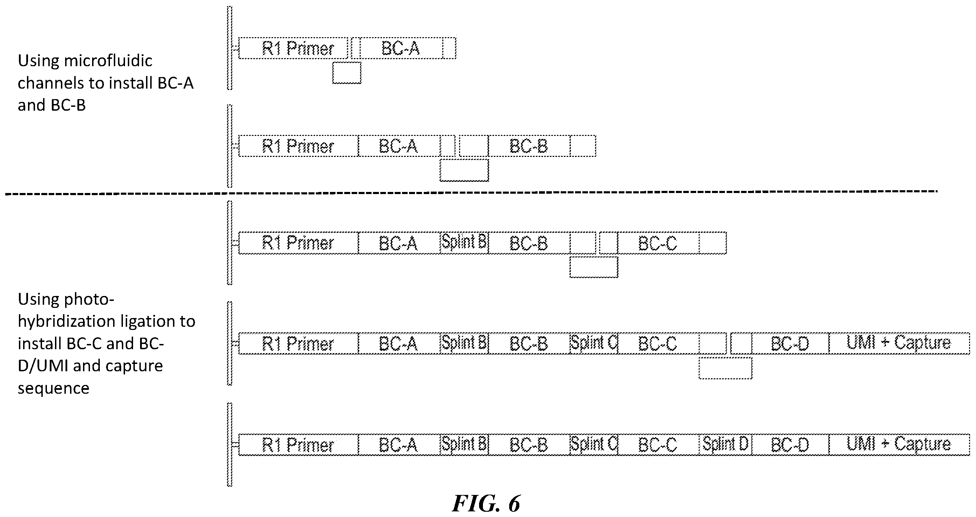

**FIG. 6** is a graph showing parts of oligonucleotides installed by microfluidic channels (top) and photo-hybridization ligation (bottom). The oligonucleotides are shown as the wider rectangles. The top portion shows an example of using microfluidic channels to install a first barcode (BC), "A" (BC-A), and then a second barcode, "B" (BC-B), to an attached oligonucleotide comprising an R1 primer (or a partial R1 primer, not shown), wherein the primer is attached to the substrate (vertical rectangle). The bottom portion shows using photo-hybridization ligation to install a third barcode, "C" (BC-C), and then a fourth barcode, ("D") (BC-D), with a unique molecular identifier (UMI) and capture sequence. Also shown are splint sequences B, C, and D. The unlabeled rectangles represent oligonucleotides that facilitate attachment, such as splints, wherein the nucleotide sequences shown between the barcodes or barcode portions (e.g., between BC-A and BC-B) comprise sequences that hybridize to a splint, which is then used as a template to attach BC-B, such that the nucleotide sequence separating BC-A and BC-B, once attached, comprises the sequence "splint B". Similarly, the portions between BC-B and BC-D comprise the "splint C" sequence once BC-C is attached, and the portions between BC-C and BC-D, comprise the "splint D" sequence once BC-D is attached.

### DETAILED DESCRIPTION

If a definition set forth herein is contrary to or otherwise inconsistent with a definition set forth in the patents, applications, published applications and other publications that are referenced, the definition set forth herein prevails over the definition that is in that reference.

The section headings used herein are for organizational purposes only and are not to be construed as limiting the subject matter described.

### I. OVERVIEW

Oligonucleotide arrays for spatial transcriptomics may be made by mechanical spotting, bead arrays, and/or *in situ* base-by-base synthesis of the oligonucleotides. In some cases, mechanical spotting is ideal for larger spot sizes (*e.g.,* 30 microns in diameter or greater), since fully elaborated oligos (*e.g*., with a desired combination and diversity of barcodes) can be spotted in a known position with high purity and fidelity. However, methods to decrease spot sizes or features at or below 10 microns (e.g., single cell scale resolution) in diameter with sufficient throughput are lacking. In some aspects, bead arrays offer a way to increase feature density. For example, barcodes are generated by first attaching an oligonucleotide to all beads and then performing multiple rounds of split-pool ligations to generate barcodes combinatorially. However, in some aspects, bead arrays result in random barcoded bead arrays that must be decoded prior to use and each array ultimately has a unique pattern. Additionally, even monodisperse beads at the 1-10 micron scale may have some variability that results in a range of feature sizes with the potential for variable oligo density.

Methods for *in situ* generated arrays have utilized photo-cleavable protecting groups to synthesize barcode oligos one nucleotide at a time. The feature size can be highly controlled using photomasks and the generated array is known and uniform across all arrays with no decoding needed. However, the oligo fidelity decreases with increasing oligo length with a ~99% per step (i.e., per nucleotide) efficiency using base-by-base *in situ* oligonucleotide synthesis. A method to overcome oligonucleotide fidelity issues is to directly ligate or hybridize entire nucleic acid sequences instead of bases onto mask-mediated photo-de-protected regions and combinatorially build barcodes on an array. Nevertheless, this approach still requires a large number of masks and long ligation cycles to fully construct all barcodes, thus reducing fidelity of alignment over the cycles.

In some aspects, provided herein are multiplexed methods and assay systems capable of high levels of multiplexing with an efficient spatial encoding scheme. In some embodiments, provided herein is a method for providing an array, wherein the array is generated using microfluidics and photolithography based ligations/hybridization. Microfluidic-guided patterning may be used as a first step of array generation, in order to generate a coarse level of barcoding. Each coarse pixel may be further patterned using photolithography to generate uniquely barcoded subpixels that grant a much higher resolution. In particular, each coarse pixel generated from microfluidic patterning can be staggered for use in photolithography.

Methods for the fabrication of patterned arrays (e.g., a substrate having coupled to it a plurality of polymer molecules, such as oligonucleotides) with high spatial resolution by microfluidics to generate a course pattern of pixels, followed by photolithography of subpixels, using caged oligonucleotides, polymers, or photoresist methodologies are also provided herein. For example, methods and uses of microfluidically controlled and light-controlled combinatorial barcode generation for molecule arrays are disclosed. In some embodiments, microfluidically controlled and light-controlled ligation for combinatorial barcode generation is utilized.

In some aspects, provided herein is a method for generating an immobilized nucleic acid, comprising: a) delivering a first oligonucleotide of at least four nucleotides in length through a first microfluidic channel to a corresponding first area on a substrate, whereby the first oligonucleotide is attached to an oligonucleotide molecule immobilized in the first area to generate an extended oligonucleotide molecule, optionally wherein the first oligonucleotide comprises a first barcode sequence; b) delivering a second oligonucleotide of at least four nucleotides in length through a second microfluidic channel to a corresponding second area on the substrate, whereby the second oligonucleotide is attached to the extended oligonucleotide molecule to generate a further extended oligonucleotide molecule in an overlapping area between the first and second areas, optionally wherein the second oligonucleotide comprises a second barcode sequence; c) irradiating the substrate to render oligonucleotide molecules in one or more regions on the substrate available for oligonucleotide attachment, whereas oligonucleotide molecules in one or more other regions on the substrate are not available for oligonucleotide attachment; and d) attaching a third oligonucleotide of at least four nucleotides in length to the further extended oligonucleotide molecule to generate an immobilized nucleic acid on the substrate, optionally wherein the third oligonucleotide comprises a third barcode sequence and the immobilized nucleic acid comprises the first, second, and third barcode sequences. Upon the attachment of the first, second, and third oligonucleotides, one or more barcode sequences are formed in the generated immobilized nucleic acid on the substrate. The formation of the one or more barcode sequences allows for the identification of the location of the immobilized nucleic acid on the substrate, e.g., through decoding of the nucleic acid array.

A method of generating an array for determining a spatial pattern of abundance, expression, and/or activity of one or more biological targets across multiple sites in a sample is provided in **FIG. 1****.** As shown in **FIG. 1**, a substrate (e.g., comprising oligonucleotide molecules immobilized on the substrate) and a microfluidic device are provided and the substrate can be affixed to the microfluidic device. A plurality of first oligonucleotides are delivered through a plurality of first microfluidic channels, that are substantially parallel to one another, to corresponding first areas on the substrate. The first oligonucleotides are attached to oligonucleotide molecules immobilized in the first areas to generate extended oligonucleotide molecules. Each of the first oligonucleotide molecules may comprise a first barcode sequence or a first part of a barcode sequence to be assembled. A plurality of second oligonucleotides are delivered through a plurality of second microfluidic channels to corresponding second areas on the substrate. The second oligonucleotides are attached to the extended oligonucleotide molecules immobilized in the overlapping areas between the first areas and the second areas to generate further extended oligonucleotide molecules. Each of the second oligonucleotide molecules may comprise a second barcode sequence or a second part of a barcode sequence to be assembled. The intersectional areas between the first and the second areas can comprise the first and second barcode sequences or the first part assembled to the second part of the barcode sequence to be assembled. The extended oligonucleotides comprising the first and second barcode sequences are blocked and unavailable for hybridization and/or ligation. By irradiation, oligonucleotide molecules in some regions are rendered available for oligonucleotide attachment, while oligonucleotide molecules in other regions remain unavailable. A plurality of third oligonucleotides are attached to the available oligonucleotide molecules via hybridization and/or ligation to further extend the molecules. Each of the third oligonucleotide molecules may comprise a third barcode sequence or a third part of a barcode sequence to be assembled. The resulting array now comprises immobilized oligonucleotide molecules comprising the first, second, and third barcode sequences, or the first, second, and third parts assembled to form the barcode sequence.

Although **FIG. 1** shows two oligonucleotide delivering steps using microfluidic channels are followed by a step of photolithography guided nucleic acid hybridization and/or ligation, it should be appreciated that various combinations of microfluidic and photolithography guided nucleic acid attachment steps can be used. For instance, a method disclosed herein can comprise delivering first oligonucleotides comprising first barcodes to first areas on the substrate through microfluidic channels, such that the first oligonucleotides can be attached to oligonucleotide molecules in the first areas. The extended oligonucleotide molecules in the first areas can be blocked (e.g., using a photoresist, photo-degradable polymer, and/or photo-caged oligonucleotides) and some of the extended oligonucleotide molecules can be selectively irradiated and rendered available for hybridization and/or ligation (e.g., using a photomask), and second oligonucleotides comprising second barcodes can be attached to the available oligonucleotides molecules via hybridization and/or ligation. The blocking and hybridization and/or ligation cycles can be repeated using different second oligonucleotides comprising different second barcodes. Then, third oligonucleotides comprising third barcodes can be delivered to the substrate to contact the further extended oligonucleotide molecules through microfluidic channels, such that the third oligonucleotides can be attached to the further extended oligonucleotide molecules. In some embodiments, an oligonucleotide comprising a barcode is attached through microfluidic channel delivery, followed by one or more steps of oligonucleotide attachment through photolithography guided nucleic acid hybridization and/or ligation. In some embodiments, an oligonucleotide comprising a barcode is attached photolithography guided nucleic acid hybridization and/or ligation, followed by one or more steps of oligonucleotide attachment through microfluidic channel delivery.

Another exemplary embodiment of the present invention is shown in **FIG. 6****,** which depicts different components of immobilized oligonucleotides that can be installed by combining microfluidics and photo-hybridization ligation. A first oligonucleotide comprising a first barcode sequence is installed (e.g., delivered) by a first microfluidic channel, and further comprises a sequence that hybridizes to a first splint, which in turn hybridizes to an immobilized oligonucleotide in a first area on a substrate (R1 primer). The first oligonucleotide is then ligated to the immobilized oligonucleotide using the first splint as a template, extending the immobilized oligonucleotide on the substrate. A second oligonucleotide comprising a second barcode sequence is installed (e.g., delivered) by a second microfluidic channel, and comprises a sequence that hybridizes to a second splint which in turn hybridizes to the extended oligonucleotide molecule in a second area on the substrate. The second oligonucleotide is then ligated to the extended oligonucleotide using the second splint as a template, further extending the immobilized oligonucleotide on the substrate, thereby generating a further extended oligonucleotide molecule in an overlapping area between the first and second areas. A third oligonucleotide comprising a third barcode sequence is installed (e.g., delivered) by photo-hybridization ligation, and comprises a sequence that hybridizes to a third splint which in turn hybridizes to the further extended oligonucleotide molecule. The third oligonucleotide is then ligated to the further extended oligonucleotide using the third splint as a template, thereby generating an even further extended immobilized oligonucleotide. Optionally, a fourth oligonucleotide comprising a fourth barcode sequence, UMI, and a capture sequence, is installed by photo-hybridization ligation, and comprises a sequence that hybridizes to a fourth splint which in turn hybridizes to the further extended oligonucleotide molecule. The fourth oligonucleotide is then ligated to the even further extended oligonucleotide using the fourth splint as a template, further extending the immobilized oligonucleotide. In any of the embodiments herein, the photo-hybridization ligation can be performed using exemplary methods and reagents described in US 2022/0228201 A1, US 2022/0228210 A1, and US 2022/0314187 A1.

The methods provided herein is advantageous in regards to generating arrays comprised of diverse barcodes in reduced time. For instance, the first and second barcodes can be incorporated by microfluidic channels, and each step can utilize multiple channels in parallel, reducing the amount of time required to pattern the whole substrate. Combining microfluidics with photo-hybridization allows further incorporating a third or fourth barcode sequence. The combination of these barcode sequences provide molecular arrays with precise spatial information that can aid spatial analyses and result in improved resolution of biological analytes.

### II. MOLECULAR ARRAYS AND MICROFLUIDICS

### A. Molecular Arrays

In some aspects, the methods provided herein comprises attaching oligonucleotides (e.g. a barcode) to a substrate. Oligonucleotides may be attached to the substrate according to the methods set forth in U.S. Patent Nos. 6,737,236, 7,259,258, 7,375,234, 7,427,678, 5,610,287, 5,807,522, 5,837,860, and 5,472,881; U.S. Patent Application Publication Nos. 2008/0280773 and 2011/0059865; Shalon et al. (1996) Genome Research, 639-645; Rogers et al. (1999) Analytical Biochemistry 266, 23-30; Stimpson et al. (1995) Proc. Natl. Acad. Sci. USA 92, 6379-6383; Beattie et al. (1995) Clin. Chem. 45, 700-706; Lamture et al. (1994) Nucleic Acids Research 22, 2121-2125; Beier et al. (1999) Nucleic Acids Research 27, 1970-1977; Joos et al. (1997) Analytical Biochemistry 247, 96-101; Nikiforov et al. (1995) Analytical Biochemistry 227, 201-209; Timofeev et al. (1996) Nucleic Acids Research 24, 3142-3148; Chrisey et al. (1996) Nucleic Acids Research 24, 3031-3039; Guo et al. (1994) Nucleic Acids Research 22, 5456-5465; Running and Urdea (1990) BioTechniques 8, 276-279; Fahy et al. (1993) Nucleic Acids Research 21, 1819-1826; Zhang *et al.* (1991) 19, 3929-3933; and Rogers et al. (1997) Gene Therapy 4, 1387-1392.

Arrays can be prepared by a variety of methods. In some embodiments, arrays are prepared through the synthesis (e.g., *in situ* synthesis) of oligonucleotides on the array, or by jet printing or lithography. For example, light-directed synthesis of high-density DNA oligonucleotides can be achieved by photolithography or solid-phase DNA synthesis. To implement photolithographic synthesis, synthetic linkers modified with photochemical protecting groups can be attached to a substrate and the photochemical protecting groups can be modified using a photolithographic mask (applied to specific areas of the substrate) and light, thereby producing an array having localized photo-deprotection. Many of these methods are known in the art, and are described e.g., in Miller et al., "Basic concepts of microarrays and potential applications in clinical microbiology." Clinical microbiology reviews 22.4 (2009): 611-633; US201314111482A; US9593365B2; US2019203275; and WO2018091676.

In any of the embodiments herein, oligonucleotide molecules on the substrate can be immobilized in a plurality of features. In any of the embodiments herein, the 3' terminal nucleotides of the immobilized oligonucleotide molecules can be distal to the substrate or array surface. In any of the embodiments herein, the 5' terminal nucleotides of the immobilized oligonucleotide molecules can be more proximal to the substrate or array surface than the 3' terminal nucleotides. In any of the embodiments herein, one or more nucleotides at or near the 5' terminus of each immobilized oligonucleotide can be directly or indirectly attached to the substrate or array surface, thereby immobilizing the oligonucleotides. In any of the embodiments herein, the 3' terminus of each immobilized oligonucleotide can project away from the substrate or array surface. In any of the embodiments herein, the 5' terminal nucleotides of the immobilized oligonucleotide molecules can be distal to the substrate or array surface. In any of the embodiments herein, the 3' terminal nucleotides of the immobilized oligonucleotide molecules can be more proximal to the substrate or array surface than the 5' terminal nucleotides. In any of the embodiments herein, one or more nucleotides at or near the 3' terminus of each immobilized oligonucleotide can be directly or indirectly attached to the substrate or array surface, thereby immobilizing the oligonucleotides. In any of the embodiments herein, the 5' terminus of each immobilized oligonucleotide can project away from the substrate or array surface.

In some embodiments, a method provided herein further comprises a step of providing the substrate. A wide variety of different substrates can be used for the foregoing purposes. In general, a substrate can be any suitable support material. The substrate may comprise materials of one or more of the IUPAC Groups 4, 6, 11, 12, 13, 14, and 15 elements, plastic material, silicon dioxide, glass, fused silica, mica, ceramic, or metals deposited on the aforementioned substrates. Exemplary substrates include, but are not limited to, glass, modified and/or functionalized glass, hydrogels, films, membranes, plastics (including e.g., acrylics, polystyrene, copolymers of styrene and other materials, polypropylene, polyethylene, polybutylene, polyurethanes, Teflon^{™}, cyclic olefins, polyimides etc.), nylon, ceramics, resins, Zeonor, silica or silica-based materials including silicon and modified silicon, carbon, quartz, metals, inorganic glasses, optical fiber bundles, and polymers, such as polystyrene, cyclic olefin copolymers (COCs), cyclic olefin polymers (COPs), polypropylene, polyethylene and polycarbonate. In some embodiments, the substrate is a glass substrate.

A substrate can be of any desired shape. For example, a substrate can be typically a thin (e.g., sub-centimeter), flat shape (e.g., square, rectangle or a circle). In some embodiments, a substrate structure has rounded corners (e.g., for increased safety or robustness). In some embodiments, a substrate structure has one or more cut-off corners (e.g., for use with a slide clamp or cross-table). In some embodiments, where a substrate structure is flat, the substrate structure can be any appropriate type of support having a flat surface (e.g., a chip, wafer, e.g., a silicon-based wafer, die, or a slide such as a microscope slide).

In some embodiments, a substrate comprising an array of molecules is provided, e.g., in the form of a lawn of polymers (e.g., oligonucleotides) on the substrate in a pattern. Examples of polymers on an array may include, but are not limited to, nucleic acids, peptides, phospholipids, polysaccharides, heteromacromolecules in which one moiety is covalently bound to any of the above, polyurethanes, polyesters, polycarbonates, polyureas, polyamides, polyethyleneimines, polyarylene sulfides, polysiloxanes, polyimides, and polyacetates. The molecules occupying different features of an array typically differ from one another, although some redundancy in which the same polymer occupies multiple features can be useful as a control. For example, in a nucleic acid array, the nucleic acid molecules within the same feature are typically the same, whereas nucleic acid molecules occupying different features are mostly different from one another.

In addition to those above, a wide variety of other features can be used to form the arrays described herein. For example, in some embodiments, features that are formed from polymers and/or biopolymers that are jet printed, screen printed, or electrostatically deposited on a substrate can be used to form arrays.

In some examples, the molecules on the array may be nucleic acids, such as oligonucleotides. The oligonucleotide can be single-stranded or double-stranded. Nucleic acid molecules on an array may be DNA or RNA. The DNA may be single-stranded or double-stranded. The DNA may include, but are not limited to, mitochondrial DNA, cell-free DNA, complementary DNA (cDNA), genomic DNA, plasmid DNA, cosmid DNA, bacterial artificial chromosome (BAC), or yeast artificial chromosome (YAC). The RNA may include, but are not limited to, mRNAs, tRNAs, snRNAs, rRNAs, retroviruses, small non-coding RNAs, microRNAs, polysomal RNAs, pre-mRNAs, intronic RNA, viral RNA, cell free RNA and fragments thereof. The non-coding RNA, or ncRNA can include snoRNAs, microRNAs, siRNAs, piRNAs and long nc RNAs.

The oligonucleotide, such as a first oligonucleotide, a second oligonucleotide, a third oligonucleotide, etc., is at least about 4 nucleotides in length, such as at least any of about 5, 6, 7, 8, 9, 10, 11, 12, 13, 14, 15, 16, 17, 18, 19, 20, 25, 30, 35, 40, 45, 50, 55, 60, 65, 70, 75, 70, or more, nucleotides in length. In some embodiments, the oligonucleotide is at least 4 nucleotides in length. In some embodiments, the oligonucleotide is less than about 70 nucleotides in length, such as less than any of about 65, 60, 55, 50, 45, 40, 35, 30, 25, 20, 19, 18, 17, 16, 15, 14, 13, 12, 11, 10, 9, 8, 7, 8, 6, 5, 4, or fewer, nucleotides in length. In some embodiments, the oligonucleotide is between about 4 and about 70 nucleotides in length, such as between about 4 and about 10 nucleotides in length, between about 5 and about 20 nucleotides in length, between about 10 and about 50 nucleotides in length, and between about 30 and about 70 nucleotides in length.

In some embodiments, the molecules on an array comprise oligonucleotide barcodes. The barcode sequences are optional. In some embodiments, a first oligonucleotide comprises a first barcode sequence. In some embodiments, a second oligonucleotide comprises a second barcode sequence. In some embodiments, a third oligonucleotide comprises a third barcode sequence. In some embodiments, the first oligonucleotide comprises a first barcode sequence but the second oligonucleotide does not comprise a barcode sequence. In some embodiments, the first oligonucleotide comprises a first barcode sequence and the second oligonucleotide comprises a second barcode sequence. In some embodiments, the first oligonucleotide comprises a first barcode sequence but the third oligonucleotide does not comprise a barcode sequence. In some embodiments, the first oligonucleotide comprises a first barcode sequence and the third oligonucleotide comprises a third barcode sequence. In some embodiments, the second oligonucleotide comprises a second barcode sequence but the third oligonucleotide does not comprise a barcode sequence. In some embodiments, the second oligonucleotide comprises a second barcode sequence and the third oligonucleotide comprises a third barcode sequence.

In some embodiments, each of the first, second, and third oligonucleotides comprise a barcode sequence (e.g., a first, second, and third barcode sequence, respectively). In some embodiments, the barcode sequence on the first oligonucleotide (e.g.,, a first barcode sequence) is different from the barcode sequence on the second oligonucleotide (e.g., a second barcode sequence). In some embodiments, the barcode sequence on the first oligonucleotide is different from the barcode sequence on the third oligonucleotide (e.g., a third barcode sequence). In some embodiments, the barcode sequence on the second oligonucleotide is different from the barcode sequence on the third oligonucleotide. In some embodiments, each of the first, second, and third oligonucleotide barcode sequences are different.

A barcode sequence can be of varied length. In some embodiments, the barcode sequence is about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 30, about 35, about 40, about 45, about 50, about 55, about 60, about 65, or about 70 nucleotides in length. In some embodiments, the barcode sequence is between about 4 and about 25 nucleotides in length. In some embodiments, the barcode sequences is between about 10 and about 50 nucleotides in length. The nucleotides can be completely contiguous, e.g., in a single contiguous stretch of adjacent nucleotides, or they can be separated into two or more separate subsequences that are separated by 1 or more nucleotides. In some embodiments, the barcode sequence can be about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or longer. In some embodiments, the barcode sequence can be at least about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or longer. In some embodiments, the barcode sequence can be at most about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or shorter.

The oligonucleotide can include one or more (e.g., two or more, three or more, four or more, five or more) Unique Molecular Identifiers (UMIs). A unique molecular identifier is a contiguous nucleic acid segment or two or more non-contiguous nucleic acid segments that function as a label or identifier for a particular analyte, or for a capture probe that binds a particular analyte (e.g., via the capture domain). A UMI can be unique. A UMI can include one or more specific polynucleotides sequences, one or more random nucleic acid and/or amino acid sequences, and/or one or more synthetic nucleic acid and/or amino acid sequences. In some embodiments, the UMI is a nucleic acid sequence that does not substantially hybridize to analyte nucleic acid molecules in a biological sample. In some embodiments, the UMI has less than 90% sequence identity (e.g., less than 80%, 70%, 60%, 50%, or less than 40% sequence identity) to the nucleic acid sequences across a substantial part (e.g., 80% or more) of the nucleic acid molecules in the biological sample.

The UMI can include from about 6 to about 20 or more nucleotides within the sequence of capture probes, e.g., barcoded oligonucleotides in an array generated using a method disclosed herein. In some embodiments, the length of a UMI sequence can be about 6, 7, 8, 9, 10, 11, 12, 13, 14, 15, 16, 17, 18, 19, 20 nucleotides or longer. In some embodiments, the length of a UMI sequence can be at least about 6, 7, 8, 9, 10, 11, 12, 13, 14, 15, 16, 17, 18, 19, 20 nucleotides or longer. In some embodiments, the length of a UMI sequence is at most about 6, 7, 8, 9, 10, 11, 12, 13, 14, 15, 16, 17, 18, 19, 20 nucleotides or shorter. These nucleotides can be contiguous, e.g., in a single stretch of adjacent nucleotides, or they can be separated into two or more separate subsequences that are separated by 1 or more nucleotides. Separated UMI subsequences can be from about 4 to about 16 nucleotides in length. In some embodiments, the UMI subsequence can be about 4, 5, 6, 7, 8, 9, 10, 11, 12, 13, 14, 15, 16 nucleotides or longer. In some embodiments, the UMI subsequence can be at least about 4, 5, 6, 7, 8, 9, 10, 11, 12, 13, 14, 15, 16 nucleotides or longer. In some embodiments, the UMI subsequence can be at most about 4, 5, 6, 7, 8, 9, 10, 11, 12, 13, 14, 15, 16 nucleotides or shorter.

In some embodiments, a UMI is attached to other parts of the nucleotide in a reversible or irreversible manner. In some embodiments, a UMI is added to, for example, a fragment of a DNA or RNA sample before, during, and/or after sequencing of the analyte. In some embodiments, a UMI allows for identification and/or quantification of individual sequencing-reads. In some embodiments, a UMI is a used as a fluorescent barcode for which fluorescently labeled oligonucleotide probes hybridize to the UMI.

In some embodiments, a method provided herein further comprises a step of providing the substrate. A wide variety of different substrates can be used for the foregoing purposes. In general, a substrate can be any suitable support material. The substrate may comprise materials of one or more of the IUPAC Groups 4, 6, 11, 12, 13, 14, and 15 elements, plastic material, silicon dioxide, glass, fused silica, mica, ceramic, or metals deposited on the aforementioned substrates. Exemplary substrates include, but are not limited to, glass, modified and/or functionalized glass, hydrogels, films, membranes, plastics (including e.g., acrylics, polystyrene, copolymers of styrene and other materials, polypropylene, polyethylene, polybutylene, polyurethanes, Teflon^{™}, cyclic olefins, polyimides etc.), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene and polycarbonate.

A substrate can be of any desired shape. For example, a substrate can be typically a thin, flat shape (e.g., a square or a rectangle). In some embodiments, a substrate structure has rounded corners (e.g., for increased safety or robustness). In some embodiments, a substrate structure has one or more cut-off corners (e.g., for use with a slide clamp or cross-table). In some embodiments, where a substrate structure is flat, the substrate structure can be any appropriate type of support having a flat surface (e.g., a chip or a slide such as a microscope slide).

### B. Methods of Microfluidically-Controlled Surface Patterning

An array generated by the methods herein (e.g., an array comprising an immobilizing nucleic acid) is first coarsely patterned using microfluidics. Microfluidics may comprise surface patterning a substrate, such as any of the substrates described herein, with one or more oligonucleotides (e.g., a first oligonucleotide and a second oligonucleotide) through a microfluidic channel (e.g., a first microfluidic channel and a second microfluidic channel) to a particular area on the substrate (e.g., a first area and a second area). The oligonucleotide may be attached to an oligonucleotide molecule immobilized in the area to generate an extended oligonucleotide molecule.

For example, the method may comprise delivering a first oligonucleotide of at least four nucleotides in length through a first microfluidic channel to a corresponding first area on a substrate, whereby the first oligonucleotide is attached to an oligonucleotide molecule immobilized in the first area to generate an extended oligonucleotide molecule. In some embodiments, the first oligonucleotide comprises a first barcode sequence. As shown in **FIG. 2A****,** a plurality of first oligonucleotide molecules are delivered to corresponding first areas via a plurality of first microfluidic channels (e.g., X1 and X2). Each of the first oligonucleotide molecules can comprise a first barcode sequence.

In some embodiments, the first oligonucleotide is at least about 4 nucleotides in length, such as at least any of about 5, 6, 7, 8, 9, 10, 11, 12, 13, 14, 15, 16, 17, 18, 19, 20, 25, 30, 35, 40, 45, 50, 55, 60, 65, 70, 75, 70, or more, nucleotides in length. In some embodiments, the first oligonucleotide is at least 4 nucleotides in length. In some embodiments, the first oligonucleotide is less than about 70 nucleotides in length, such as less than any of about 65, 60, 55, 50, 45, 40, 35, 30, 25, 20, 19, 18, 17, 16, 15, 14, 13, 12, 11, 10, 9, 8, 7, 8, 6, 5, 4, or fewer, nucleotides in length. In some embodiments, the first oligonucleotide is between about 4 and about 70 nucleotides in length, such as between about 4 and about 10 nucleotides in length, between about 5 and about 20 nucleotides in length, between about 10 and about 50 nucleotides in length, and between about 30 and about 70 nucleotides in length.

In some embodiments, the first oligonucleotide comprises a sequence that hybridizes to a first splint, which in turn hybridizes to the oligonucleotide molecule (e.g., the oligonucleotide molecule in the first area). In some embodiments, the sequence of the first oligonucleotide that hybridizes to the first splint is a portion of the first oligonucleotide. In some embodiments, the first oligonucleotide is ligated to the oligonucleotide molecule using the first splint as a template to generate the extended oligonucleotide molecule. In some embodiments, the ligation is a splint templated ligation. In some embodiments, the splint templated ligation generates the extended oligonucleotide molecule. In some embodiments, gaps between the first oligonucleotide and the oligonucleotide molecule may first be filled prior to ligation, using, for example, Mu polymerase, DNA polymerase, RNA polymerase, reverse transcriptase, VENT polymerase, *Taq* polymerase, and/or any combinations, derivatives, and variants (e.g., engineered mutants) thereof.

The method may further comprise delivering a second oligonucleotide of at least four nucleotides in length through a second microfluidic channel to a corresponding second area on a substrate, whereby the second oligonucleotide is attached to an oligonucleotide molecule immobilized in the second area to generate an extended oligonucleotide molecule. In some embodiments, the second oligonucleotide comprises a second barcode sequence. As shown in **FIG. 2B****,** a plurality of second oligonucleotide molecules are delivered to corresponding second areas via a plurality of second microfluidic channels (e.g., Y1 and Y2). Each of the second oligonucleotide molecules can comprise a second barcode sequence.

In some embodiments, the second oligonucleotide is at least about 4 nucleotides in length, such as at least any of about 5, 6, 7, 8, 9, 10, 11, 12, 13, 14, 15, 16, 17, 18, 19, 20, 25, 30, 35, 40, 45, 50, 55, 60, 65, 70, 75, 70, or more, nucleotides in length. In some embodiments, the second oligonucleotide is at least 4 nucleotides in length. In some embodiments, the second oligonucleotide is less than about 70 nucleotides in length, such as less than any of about 65, 60, 55, 50, 45, 40, 35, 30, 25, 20, 19, 18, 17, 16, 15, 14, 13, 12, 11, 10, 9, 8, 7, 8, 6, 5, 4, or fewer, nucleotides in length. In some embodiments, the second oligonucleotide is between about 4 and about 70 nucleotides in length, such as between about 4 and about 10 nucleotides in length, between about 5 and about 20 nucleotides in length, between about 10 and about 50 nucleotides in length, and between about 30 and about 70 nucleotides in length.

In some embodiments, the second oligonucleotide comprises a sequence that hybridizes to a second splint, which in turn hybridizes to the oligonucleotide molecule (e.g., the oligonucleotide molecule in the second area). In some embodiments, the sequence of the second oligonucleotide that hybridizes to the second splint is a portion of the second oligonucleotide. In some embodiments, the second oligonucleotide is ligated to the oligonucleotide molecule using the second splint as a template to generate the further extended oligonucleotide molecule. In some embodiments, the ligation is a splint templated ligation. In some embodiments, the splint templated ligation generates the further extended oligonucleotide molecule. In some embodiments, gaps between the second oligonucleotide and the extended oligonucleotide molecule may first be filled prior to ligation, using, for example, Mu polymerase, DNA polymerase, RNA polymerase, reverse transcriptase, VENT polymerase, *Taq* polymerase, and/or any combinations, derivatives, and variants (e.g., engineered mutants) thereof.

As shown in **FIG. 2C****,** the combination of the first barcode sequence and the second barcode sequence can be used to locate the overlapping areas between the first area and the second area (e.g., X1 and Y1; X1 and Y2; X2 and Y1; or X2 and Y2), therefore providing spatial information of the oligonucleotide molecules immobilized on the array.

In some embodiments, the first microfluidic channel is a different microfluidic channel from the second microfluidic channel. In some embodiments, the first and second microfluidic channels are provided in the same microfluidic device. In some embodiments, the first and second microfluidic channels are provided in different microfluidic devices. As shown in **FIG. 3****,** the first and/or the second microfluidic channels can comprise a plurality of microfluidic channels that are substantially parallel to another. Each step can utilize a subset of the plurality of microfluidic channels to deliver oligonucleotides, therefore reducing the amount of time required to pattern the array. The oligonucleotides delivered by each channel in a given step can be different from the oligonucleotides delivered by the other channels.

In some embodiments, the relative positions of the first and second microfluidic channels are described in terms of an angle between the first and second microfluidic channels. In some embodiments, the angle between the first and second microfluidic channels is between about 10 degrees and about 90 degrees, such as between about 10 degrees and about 40 degrees, between about 20 degrees and about 60 degrees, between about 30 degrees and about 80 degrees, and between about 50 degrees and about 90 degrees. In some embodiments, the angle between the first and second microfluidic channels is less than about 90 degrees, such as less than any of about 80 degrees, 70 degrees, 60 degrees, 50 degrees, 40 degrees, 30 degrees, 20 degrees, 10 degrees, or less. In some embodiments, the angle between the first and second microfluidic channels is greater than about 10 degrees, such as greater than any of about 20 degrees, 30 degrees, 40 degrees, 50 degrees, 60 degrees, 70 degrees, 80 degrees, 90 degrees, or greater. In some embodiments, the angle between the first and second microfluidic channels is about 90 degrees, about 80 degrees, about 70 degrees, about 60 degrees, about 50 degrees, about 40 degrees, about 30 degrees, about 20 degrees, or about 10 degrees.

In some embodiments, the width of the first and/or second microfluidic channels is between about 5 µm and about 500 µm, such as between about 5 µm and about 100 µm, between about 50 µm and about 300 µm, and between about 250 µm and about 500 µm. In some embodiments, the width of the first and/or second microfluidic channels is less than about 500 µm, such as less than any of about 450 µm, 400 µm, 350 µm, 300 µm, 250 µm, 200 µm, 150 µm, 100 µm, 50 µm, 10 µm, 5 µm, or less. In some embodiments, the width of the first and/or second microfluidic channels is greater than about 5 µm, such as greater than any of about 10 µm, 50 µm, 100 µm, 150 µm, 200 µm, 250 µm, 300 µm, 350 µm, 400 µm, 450 µm, 500 µm, or greater. In some embodiments, the width of the first and/or second microfluidic channels is about 5 µm, about 10 µm, about 50 µm, about 100 µm, about 150 µm, about 200 µm, about 250 µm, about 300 µm, about 350 µm, about 400 µm, about 450 µm, or about 500 µm.

In some embodiments, the depth of the first and/or second microfluidic channels is between about 5 µm and about 500 µm, such as between about 5 µm and about 100 µm, between about 50 µm and about 300 µm, and between about 250 µm and about 500 µm. In some embodiments, the depth of the first and/or second microfluidic channels is less than about 500 µm, such as less than any of about 450 µm, 400 µm, 350 µm, 300 µm, 250 µm, 200 µm, 150 µm, 100 µm, 50 µm, 10 µm, 5 µm, or less. In some embodiments, the depth of the first and/or second microfluidic channels is greater than about 5 µm, such as greater than any of about 10 µm, 50 µm, 100 µm, 150 µm, 200 µm, 250 µm, 300 µm, 350 µm, 400 µm, 450 µm, 500 µm, or greater. In some embodiments, the depth of the first and/or second microfluidic channels is about 5 µm, about 10 µm, about 50 µm, about 100 µm, about 150 µm, about 200 µm, about 250 µm, about 300 µm, about 350 µm, about 400 µm, about 450 µm, or about 500 µm.

The methods of generating an oligonucleotide array provided herein may be multiplexed. For example, in some aspects, the method may comprise delivering a plurality of first oligonucleotides each comprising a first barcode sequence through a plurality of first microfluidic channels to corresponding first areas on a substrate, whereby the plurality of first oligonucleotides are attached to oligonucleotide molecules immobilized in the first areas to generate extended oligonucleotide molecules. In some embodiments, the method further comprises delivering a plurality of second oligonucleotides each comprising a second barcode sequence through a plurality of second microfluidic channels to corresponding second areas on the substrate, whereby the plurality of second oligonucleotides are attached to the extended oligonucleotide molecules to generate further extended oligonucleotide molecules in overlapping areas between the first areas and the second areas.

In some embodiments, the plurality of first microfluidic channels are substantially parallel to each other. In some embodiments, the plurality of second microfluidic channels are substantially parallel to each other.

In some embodiments, the number of the first and/or second microfluidic channels is between about 100 and about 1000, such as between about 100 and about 150, between about 150 and about 200, between about 200 and about 250, between about 250 and about 300, between about 300 and about 350, between about 350 and about 400, between about 400 and about 450, between about 450 and about 500, between about 500 and about 550, between about 550 and about 600, between about 600 and about 650, between about 650 and about 700, between about 700 and about 750, between about 750 and about 800, between about 800 and about 850, between about 850 and about 900, between about 900 and about 950, and between about 950 and about 1000. In some embodiments, the number of the first and/or second microfluidic channels is less than about 1000, such as less than any of about 950, 900, 850, 800, 750, 700, 650, 600, 550, 500, 450, 400, 350, 300, 250, 200, 150, 100, or less. In some embodiments, the number of the first and/or second microfluidic channels is greater than about 100, such as greater than any of about 150, 200, 250, 300, 350, 400, 450, 500, 550, 600, 650, 700, 750, 800, 850, 900, 950, 1000, or greater.

In some embodiments, the distance between each first microfluidic channel and/or between each second microfluidic channel is between about 5 µm and about 2.00 mm, such as between about 5 µm and about 100 µm, between about 50 µm and about 300 µm, between about 200 µm and about 500 µm, between about 400 µm and about 1.0 mm, between about 800 µm and about 1.5 mm, and between about 1.0 mm and about 2.0 mm. In some embodiments, the distance between each first microfluidic channel and/or between each second microfluidic channel is less than about 2.0 mm, such as less than any of about 1.9 mm, 1.8 mm, 1.7 mm, 1.6 mm, 1.5 mm, 1.4 mm, 1.3 mm, 1.2 mm, 1.1 mm, 1.0 mm, 950 µm, 900 µm, 850 µm, 800 µm, 750 µm, 700 µm, 650 µm, 600 µm, 550 µm, 500 µm, 450 µm, 400 µm, 350 µm, 300 µm, 250 µm, 200 µm, 150 µm, 100 µm, 50 µm, 10 µm, 5 µm, or less. In some embodiments, the distance between each first microfluidic channel and/or between each second microfluidic channel is greater than about 5 µm, such as great than any of about 10 µm, 50 µm, 100 µm, 150 µm, 200 µm, 250 µm, 300 µm, 350 µm, 400 µm, 450 µm, 500 µm, 550 µm, 600 µm, 650 µm, 700 µm, 750 µm, 800 µm, 850 µm, 900 µm, 950 µm, 1.0 mm, 1.1 mm, 1.2 mm, 1.3 mm, 1.4 mm, 1.5 mm, 1.6 mm, 1.7 mm, 1.8 mm, 1.9 mm, 2.0 mm, or greater. In some embodiments, the distance between each first microfluidic channel and/or between each second microfluidic channel is about 5 µm, about 10 µm, about 50 µm, about 100 µm, about 150 µm, about 200 µm, about 250 µm, about 300 µm, about 350 µm, about 400 µm, about 450 µm, about 500 µm, about 550 µm, about 600 µm, about 650 µm, about 700 µm, about 750 µm, about 800 µm, about 850 µm, about 900 µm, about 950 µm, about 1.0 mm, about 1.2 mm, about 1.3 mm, about 1.4 mm, about 1.5 mm, about 1.6 mm, about 1.7 mm, about 1.8 mm, about 1.9 mm, or about 2.0 mm.

In some embodiments, the delivery of first and/or second oligonucleotides comprises multiple steps. In some embodiments, the delivery of first oligonucleotides comprises multiple steps. In some embodiments, in each step a subset of the plurality of first oligonucleotides are delivered to the corresponding first areas on the substrate. In some embodiments, the first microfluidic channels are reused for delivery in the multiple steps. In some embodiments, the delivery of second oligonucleotides comprises multiple steps. In some embodiments, in each step a subset of the plurality of second oligonucleotides are delivered to the corresponding second areas on the substrate. In some embodiments, the second microfluidic channels are reused for delivery in the multiple steps.

In some embodiments, the delivery system is a flow-based microfluidic system. The flow-based microfluidic systems for oligonucleotide (e.g., first and/or second oligonucleotide) delivery in the present invention can include instrumentation such as one or more pumps, valves, fluid reservoirs, channels, and/or reagent storage cells. In some embodiments, the microfluidics system moves fluid in order to contact a discrete section of the substrate, for example a substrate comprising a biological sample. In some embodiments, a pump is used to deliver the oligonucleotides to the substrate. In some embodiments, the oligonucleotides are delivered to the substrate by gravity.

### III. PHOTOLITHOGRAPHY AND LIGHT-CONTROLLED SURFACE PATTERNING

Provided herein in some embodiments are methods and uses of photo-hybridization-ligation combinatorial barcode generation using any suitable light-controlled surface patterning (e.g., photoresist, polymers, caged oligonucleotides, etc.) and photolithography. For example, a method disclosed herein may comprise photocontrollable ligation, wherein local irradiation causes degradation of photoresist and oligonucleotides to be exposed for ligation. In some aspects, a method disclosed herein provides one or more advantages as compared to available arraying methods. For example, a large diversity of barcodes can be created via sequential rounds of UV exposure, hybridization, ligation, removal and reapplication using light-controlled surface patterning; no protection/deprotection step is required for ligating oligonucleotides to the substrate; the feature size can be highly controlled using photomasks, and the generated array at any discrete location is known and across all arrays with no decoding needed.

The methods of the present disclosure comprise irradiating a substrate to render oligonucleotide molecules in one or more regions on the substrate available for oligonucleotide attachment, whereas oligonucleotide molecules in one or more other regions on the substrate are not available for oligonucleotide attachment. In some embodiments, the irradiation is selective, for example, where one or more photomasks can be used such that only one or more specific regions of the array are exposed to stimuli (*e.g*., exposure to light such as UV, and/or exposure to heat induced by laser). In some embodiments, the method comprises irradiating oligonucleotide molecules in one or more regions with a first light while oligonucleotide molecules in one or more other regions are not irradiated with the first light. For instance, the substrate is exposed to the first light when the oligonucleotide molecules in the one or more other regions are photomasked while the oligonucleotide molecules in the one or more regions are not photomasked. Alternatively, a focused light such as laser may be used to irradiate the oligonucleotide molecules in the one or more regions but not the oligonucleotide molecules in the one or more other regions, even when the oligonucleotide molecules in the one or more other regions are not masked from the light. For example, the distance (pitch) between features may be selected to prevent the laser from stimulating oligonucleotides of an adjacent feature.

In some embodiments, during and after the irradiation, the oligonucleotide molecules in the one or more other regions are protected from hybridization (e.g., hybridization to a splint and/or an oligonucleotide molecule). In some embodiments, during and after the irradiation, the oligonucleotide molecules in the one or more other regions are protected from ligation (e.g., hybridization to a splint and/or an oligonucleotide molecule). In some embodiments, during and after the irradiation, the oligonucleotide molecules in the one or more other regions are protected from hybridization and ligation. Various strategies for the protection of the oligonucleotides from hybridization and/or ligation are contemplated herein.

**FIG. 4** shows oligonucleotide molecules on an array generated by microfluidic channels can be further extended using photo-hybridization ligation. The oligonucleotide molecules are first blocked and unavailable for hybridization and/or ligation, using methods such as photo-cleavable polymers, photo-cleavable moieties, and photoresist. The substrate is then irradiated selectively, rendering the oligonucleotide molecules (e.g., the further extended oligonucleotide molecules comprising a first oligonucleotide and a second oligonucleotide attached to an oligonucleotide molecule on a substrate) in some regions available for hybridization and/or ligation (i.e., deblocked). The deblocked oligonucleotide molecules are then further extended via hybridization and/or ligation, while the oligonucleotide molecules in the other regions remain blocked.

The irradiating may be multiplexed. In some embodiments, the method comprises irradiating the substrate in multiple cycles (**FIG. 5**). For instance, each cycle of irradiating may comprise irradiating one or more regions on the substrate that are different from the one or more regions irradiated in another cycle. In some embodiments, the method comprises irradiating the overlapping area between a first area and second area (e.g., on the substrate) in multiple cycles, each cycle for irradiating one or more regions in the overlapping area that are different from the region(s) irradiated in another cycle. In some embodiments, the method further comprises translating the photomask from a first position to a second position relative to the substrate, each position for a cycle of irradiating the substrate. For example, **FIG. 5** shows that a particular overlapping area between the first areas and the second areas is further patterned by N cycles and M rounds of irradiation and hybridization/ligation to generate further barcode diversity.

In some aspects, following the irradiation, the method comprises attaching an third oligonucleotide of at least four nucleotides in length to the further extended oligonucleotide molecule to generate an immobilized nucleic acid on the substrate. In some embodiments, the third oligonucleotide comprises a third barcode sequence. Thus, in some embodiments, the immobilized nucleic acid comprises the first, second, and third barcode sequences.

In some embodiments, the third oligonucleotide comprises a sequence that hybridizes to a third splint which in turn hybridizes to the further extended oligonucleotide molecule (e.g., the oligonucleotide molecule in overlapping area between the first are and the second area). In some embodiments, the sequence of the third oligonucleotide that hybridizes to the third splint is a portion of the second oligonucleotide. In some embodiments, the third oligonucleotide is ligated to the further extended oligonucleotide molecule using the third splint as a template to generate the immobilized oligonucleotide molecule. In some embodiments, the ligation is a splint templated ligation. In some embodiments, the splint templated ligation generates the immobilized oligonucleotide molecule. In some embodiments, gaps between the third oligonucleotide and the further extended oligonucleotide molecule may first be filled prior to ligation, using, for example, Mu polymerase, DNA polymerase, RNA polymerase, reverse transcriptase, VENT polymerase, *Taq* polymerase, and/or any combinations, derivatives, and variants (e.g., engineered mutants) thereof.

In some embodiments, the third oligonucleotide is at least about 4 nucleotides in length, such as at least any of about 5, 6, 7, 8, 9, 10, 11, 12, 13, 14, 15, 16, 17, 18, 19, 20, 25, 30, 35, 40, 45, 50, 55, 60, 65, 70, 75, 70, or more, nucleotides in length. In some embodiments, the third oligonucleotide is at least 4 nucleotides in length. In some embodiments, the third oligonucleotide is less than about 70 nucleotides in length, such as less than any of about 65, 60, 55, 50, 45, 40, 35, 30, 25, 20, 19, 18, 17, 16, 15, 14, 13, 12, 11, 10, 9, 8, 7, 8, 6, 5, 4, or fewer, nucleotides in length. In some embodiments, the third oligonucleotide is between about 4 and about 70 nucleotides in length, such as between about 4 and about 10 nucleotides in length, between about 5 and about 20 nucleotides in length, between about 10 and about 50 nucleotides in length, and between about 30 and about 70 nucleotides in length.

As described herein, the immobilized nucleic acid generated using a method provided herein can provide a higher resolution and/or higher barcode accuracy compared to certain methods of light mediated base-by-base *in situ* synthesis.

### A. Photoresist

In some embodiments, a method disclosed herein comprises (a) irradiating a substrate comprising an unmasked first region and a masked second region, whereby a photoresist in the first region is degraded to render oligonucleotide molecules in the first region available for hybridization and/or ligation, whereas oligonucleotide molecules in the second region are protected by a photoresist in the second region from hybridization and/or ligation; and (b) attaching an oligonucleotide comprising a barcode sequence to oligonucleotide molecules in the first region via hybridization and/or ligation, wherein oligonucleotide molecules in the second region do not receive the barcode sequence, thereby providing on the substrate an array comprising different oligonucleotide molecules in the first and second regions.

In some embodiments, oligonucleotide molecules on the substrate comprise one or more common sequences. In some embodiments, oligonucleotide molecules on the substrate comprise functional groups. In some embodiments, the functional groups are not protected by a photo-sensitive moiety prior to the irradiating step. In some embodiments, the functional groups are 3' hydroxyl groups of nucleotides. In some embodiments, the method further comprises forming a pattern of oligonucleotide molecules on the substrate prior to applying the photoresist to the substrate. In some embodiments, forming the pattern of oligonucleotide molecules comprises: irradiating a substrate comprising a plurality of functional groups and a photoresist through a patterned mask, whereby the photoresist in a first region of the substrate is degraded, rendering functional groups in the first region available for reacting with functional groups in functionalized oligonucleotide molecules, whereas functional groups in a second region of the substrate are protected by the photoresist from reacting with functional groups in the oligonucleotide molecules; and contacting the substrate with the functionalized oligonucleotide molecules, wherein the functionalized oligonucleotide molecules are coupled to functional groups in the first region but not to functional groups in the second region, thereby forming a pattern of oligonucleotide molecules on the substrate. In some embodiments, the functional groups in the functionalized oligonucleotide molecules are amino groups. In some embodiments, the method further comprises rendering the reaction between functional groups of the substrate and the functionalized oligonucleotide molecules irreversible. In some embodiments, the irradiating and contacting steps are repeated in one or more cycles. In some embodiments, the photoresist is not removed prior to, during, or between the one or more cycles. In some embodiments, the substrate is irradiated through a patterned mask. In some embodiments, the method comprises removing the patterned mask after the irradiating step, wherein the same patterned mask is re-used in a subsequent cycle of the irradiating and contacting steps. In some embodiments, the photoresist in the first region of the substrate is dissolved by a developer and removed. In some embodiments, the barcode sequence is between about 4 and about 25 nucleotides in length. In some embodiments, the oligonucleotide comprising the barcode sequence is between about 10 and about 50 nucleotides in length. In some embodiments, the oligonucleotide comprising the barcode sequence is hybridized to an oligonucleotide molecule in the first region. In some embodiments, the oligonucleotide comprising the barcode sequence is ligated to an oligonucleotide molecule in the first region. In some embodiments, the oligonucleotide comprising the barcode sequence is hybridized to a splint which is in turn hybridized to an oligonucleotide molecule in the first region. In some embodiments, the method further comprises ligating the oligonucleotide comprising the barcode sequence to the oligonucleotide molecule to generate a barcoded oligonucleotide molecule in the first region. In some embodiments, the method further comprises blocking the 3' or 5' termini of barcoded oligonucleotide molecules and/or unligated oligonucleotide molecules in the first region from ligation. In some embodiments, the photoresist is not removed prior to, during, or between any of the cycles. In some embodiments, the feature is no more than 10 microns in diameter.

In some embodiments, a method disclosed herein comprises (a) irradiating a substrate comprising an unmasked first region and a masked second region, whereby a photoresist in the first region is degraded to render oligonucleotide molecules in the first region available for hybridization and/or ligation, whereas oligonucleotide molecules in the second region are protected by the photoresist in the second region from hybridization and/or ligation; and (b) contacting oligonucleotide molecules in the first region with a first splint and a first oligonucleotide comprising a first barcode sequence, wherein the first splint hybridizes to the first oligonucleotide and the oligonucleotide molecules in the first region, wherein the first oligonucleotide is ligated to the oligonucleotide molecules in the first region, and the first oligonucleotide is not ligated to oligonucleotide molecules in the second region, thereby providing on the substrate an array comprising different oligonucleotide molecules in the first and second regions. In some embodiments, the photoresist is a first photoresist, and the first oligonucleotide is ligated to the oligonucleotide molecules in the first region to generate first extended oligonucleotide molecules, and the method further comprises: (c) applying a second photoresist to the substrate, optionally wherein the second photoresist is applied after the first photoresist is removed from the substrate; (d) irradiating the substrate while the first region is masked and the second region is unmasked, whereby the first or second photoresist in the second region is degraded to render oligonucleotide molecules in the second region available for hybridization and/or ligation, whereas the first extended oligonucleotide molecules in the first region are protected by the second photoresist in the first region from hybridization and/or ligation; and (e) contacting oligonucleotide molecules in the second region with a second splint and a second oligonucleotide comprising a second barcode sequence, wherein the second splint hybridizes to the second oligonucleotide and the oligonucleotide molecules in the second region, wherein the second oligonucleotide is ligated to the oligonucleotide molecules in the second region to generate second extended oligonucleotide molecules, and the second oligonucleotide is not ligated to the first extended oligonucleotide molecules in the first region.

In any of the preceding embodiments, the unmasked first region and the masked second region can be in an overlapping area between the first and second areas for microfluidic channel-guided oligonucleotide delivery and attachments. For instance, the unmasked first region and the masked second region can be in an intersectional area as shown in FIG. 2C.

In some embodiments, the oligonucleotide molecules are protected from hybridization by a photoresist covering the oligonucleotide molecules. In some embodiments, the oligonucleotide molecules are protected from ligation by a photoresist covering the oligonucleotide molecules. In some embodiments, the oligonucleotide molecules are protected from hybridization and ligation by a photoresist covering the oligonucleotide molecules. In some embodiments, the photoresist in irradiated regions is removed. In some embodiments, the photoresist in masked or non-irradiated regions is not removed.

A photoresist is a light-sensitive material used in processes (such as photolithography and photoengraving) to form a pattern on a surface. A photoresist may comprise a polymer, a sensitizer, and/or a solvent. The photoresist composition used herein is not limited to any specific proportions of the various components. Photoresists can be classified as positive or negative. In positive photoresists, the photochemical reaction that occurs during light exposure weakens the polymer, making it more soluble to developer, so a positive pattern is achieved. In the case of negative photoresists, exposure to light causes polymerization of the photoresist, and therefore the negative photoresist remains on the surface of the substrate where it is exposed, and the developer solution removes only the unexposed areas. In some embodiments, the photoresist used herein is a negative photoresist. In some embodiments, the photoresist used herein is a positive photoresist. In some embodiments, the photoresist is removable with UV light.

The photoresist may experience changes in pH upon irradiation. In some embodiments, the photoresist in one or more regions comprises a photoacid generator (PAG). In some embodiments, the photoresist in one or more other regions comprises a PAG. In some embodiments, the photoresist in the one or more regions and the one or more other regions comprises a PAG. In some embodiments, the photoresist in the one or more regions and the one or more other regions comprises the same PAG. In some embodiments, the photoresist in the one or more regions and the one or more other regions comprises different PAG. In some embodiments, the PAG or PAGs irreversibly release protons upon absorption of light. PAGs may be used as components of photocurable polymer formulations and chemically amplified photoresists. Examples of PAGs include triphenylsulfonium triflate, diphenylsulfonium triflate, diphenyliodonium nitrate, N-Hydroxynaphthalimide triflate, triarylsulfonium hexafluorophosphate salts, N-hydroxy-5-norbornene-2,3-dicarboximide perfluoro-1-butanesulfonate, bis(4-tert-butylphenyl)iodonium perfluoro-1-butanesulfonate, etc.

In some embodiments, the photoresist further comprises an acid scavenger. In some embodiments, the photoresist in the one or more regions and the one or more other regions comprises the same acid scavenger. In some embodiments, the photoresist in the one or more regions and the one or more other regions comprises different acid scavengers. In some embodiments, an acid scavenger acts to neutralize, adsorb and/or buffer acids, and may comprise a base or alkaline compound. In some embodiments, acid scavengers act to reduce the amount or concentration of protons or protonated water. In some embodiments, an acid scavenger acts to neutralize, diminish, or buffer acid produced by a PAG. In some embodiments,
an acid scavenger exhibits little or no stratification over time or following exposure to heat. In some embodiments, acid scavengers may be further subdivided into "organic bases" and "polymeric bases." A polymeric base is an acid scavenger (e.g., basic unit) attached to a longer polymeric unit. A polymer is typically composed of a number of coupled or linked monomers. The monomers can be the same (to form a homopolymer) or different (to form a copolymer). In a polymeric base, at least some of the monomers act as acid scavengers. An organic base is a base which is joined to or part of a non-polymeric unit. Non-limiting examples of organic bases include, without limitation, amine compounds (e.g., primary, secondary and tertiary amines). Generally any type of acid scavenger, defined here as a traditional Lewis Base, an electron pair donor, can be used in accordance with the present disclosure.

In some embodiments, the photoresist further comprises a base quencher. Base quenchers may be used in photoresist formulations to improve performance by quenching reactions of photoacids that diffuse into unexposed regions. Base quenchers may comprise aliphatic amines, aromatic amines, carboxylates, hydroxides, or combinations thereof. Examples of base quenchers include but are not limited to, trioctylamine, 1,8-diazabicyclo[5.4.0]undec-7-ene (DBU), 1-piperidineethanol (1PE), tetrabutylammonium hydroxide (TBAH), dimethylamino pyridine, 7-diethylamino-4-methyl coumarin (Coumarin 1), tertiary amines, sterically hindered diamine and guanidine bases such as 1,8-bis(dimethylamino)naphthalene (PROTON SPONGE), berberine, or polymeric amines such as in the PLURONIC or TETRONIC series commercially available from BASF. In some embodiments, the photoresist in the one or more regions and the one or more other regions comprises the same base quencher. In some embodiments, the photoresist in the one or more regions and the one or more other regions comprises different base quenchers.

In some embodiments, the photoresist further comprises a photosensitizer. A photosensitizer is a molecule that produces a chemical change in another molecule in a photochemical process. Photosensitizers are commonly used in polymer chemistry in reactions such as photopolymerization, photocrosslinking, and photodegradation. Photosensitizers generally act by absorbing ultraviolet or visible region of electromagnetic radiation and transferring it to adjacent molecules. In some embodiments, photosensitizer shifts the photo sensitivity to a longer wavelength of electromagnetic radiation. The sensitizer, also called a photosensitizer, is capable of activating the PAG at, for example, a longer wavelength of light in accordance with an aspect of the present invention. Preferably, the concentration of the sensitizer is greater than that of the PAG, such as 1.1 times to 5 times greater, for example, 1.1 times to 3 times greater the concentration of PAG. Exemplary sensitizers suitable for use in the invention include but are not limited to, isopropylthioxanthone (ITX) and 10H-phenoxazine (PhX). In some embodiments, the photoresist in the one or more regions and the one or more other regions comprises the same photosensitizer. In some embodiments, the photoresist in the one or more regions and the one or more other regions comprises different photosensitizers.

In some embodiments, the photoresist further comprises a matrix. The matrix generally refers to polymeric materials that may provide sufficient adhesion to the substrate when the photoresist formulation is applied to the top surface of the substrate, and may form a substantially uniform film when dissolved in a solvent and spread on top of a substrate. Examples of a matrix may include, but are not limited to, polyester, polyimide, polyethylene naphthalate (PEN), polyvinyl chloride (PVC), polymethylmethacrylate (PMMA), polyglycidalmethacrylate (PGMA), and polycarbonate, or a combination thereof. The matrix may be chosen based on the wavelength of the radiation used for the generation of acid when using the photoresist formulation, the adhesion properties of the matrix to the top surface of the substrate, the compatibility of the matrix to other components of the formulation, and the ease of removable or degradation (if needed) after use. In some embodiments, the photoresist in the one or more regions and the one or more other regions comprises the same matrix. In some embodiments, the photoresist in the one or more regions and the one or more other regions comprises different matrices.

In some embodiments, the photoresist further comprises a surfactant. Surfactants may be used to improve coating uniformity, and may include ionic, non-ionic, monomeric, oligomeric, and polymeric species, or combinations thereof. Examples of possible surfactants include fluorine-containing surfactants such as the FLUORAD series available from 3M Company in St. Paul, Minn., and siloxane-containing surfactants such as the SILWET series available from Union Carbide Corporation in Danbury, Conn. In some embodiments, the photoresist in the one or more regions and the one or more other regions comprises the same surfactant. In some embodiments, the photoresist in the one or more regions and the one or more other regions comprises different surfactants.

In some embodiments, the photoresist further comprises a casting solvent. A casting solvent may be used so that the photoresist may be applied evenly on the substrate surface to provide a defect-free coating. Examples of suitable casting solvents may include ethers, glycol ethers, aromatic hydrocarbons, ketones, esters, ethyl lactate, γ-butyrolactone, cyclohexanone, ethoxyethylpropionate (EEP), a combination of EEP and gamma-butyrolactone (GBL), and propylene glycol methyl ether acetate (PGMEA). In some embodiments, the photoresist in the one or more regions and the one or more other regions comprises the same casting solvent. In some embodiments, the photoresist in the one or more regions and the one or more other regions comprises different casting solvents.

Methods of applying photoresist to the substrate include, but are not limited to, dipping, spreading, spraying, or any combination thereof. In some embodiments, the photoresist is applied via spin coating, thereby forming a photoresist layer on the substrate.

In some embodiments, the photoresist is in direct contact with the oligonucleotides on the substrate. In some embodiments, the oligonucleotide molecules on the substrate are embedded in the photoresist. In some embodiments, the photoresist is not in direct contact with the oligonucleotides. In some embodiments, oligonucleotide molecules on the substrate are embedded in an underlayer that is underneath the photoresist. For example, oligonucleotide molecules on the substrate may be embedded in a soluble polymer underlayer (e.g., a soluble polyimide underlayer (XU-218)), and the photoresist forms a photoresist layer on top of the underlayer.

In some embodiments, the photoresist may be removed and re-applied for one or more times. For example, the photoresist may be stripped from the substrate and/or the oligonucleotides ligated to the substrate. Removal of photoresist can be accomplished with various degrees of effectiveness. In some embodiments, the photoresist is completely removed from the substrate and/or the oligonucleotides ligated to the substrate before re-application. Methods of removing photoresist may include, but are not limited to, using organic solvent mixtures, using liquid chemicals, exposure to a plasma environment, or other dry techniques such as UV/O₃ exposure. In some embodiments, the photoresist is stripped using organic solvent.

In some embodiments, one or more photomasks may be used to selectively remove photoresist on the substrate. The mask is designed in such a way that the exposure sites can be selected, and thus specify the coordinates on the array where each oligonucleotide can be attached. The process can be repeated, a new mask is applied activating different sets of sites and coupling different barcodes, allowing oligonucleotide molecules to be constructed at each site. This process can be used to synthesize hundreds of thousands or millions of different oligonucleotides. In some embodiments, the substrate is irradiated through a patterned mask. The mask may be an opaque plate or film with transparent areas that allow light to shine through in a pre-defined pattern. After the irradiation step, the mask may be removed, translated to a different region on the substrate, or rotated. In some embodiments, a different photomasking pattern may be used in each barcoding round. In some embodiments, the same photomasking pattern may be used in each barcoding round. Using a series of photomasks, photoresist in desired regions of the substrate may be iteratively irradiated and subsequently removed.

The material of the photomask used herein may comprise silica with chrome in the opaque part. For example, the photomask may be transparent fused silica blanks covered with a pattern defined with a chrome metal absorbing film. The photomask may be used at various irradiation wavelengths, which include but are not limited to, 365 nm, 248 nm, and 193 nm. In some embodiments, the irradiation step herein can be performed for a duration of between about 1 minute and about 10 minutes, for example, for about 2 minutes, about 4 minutes, about 6 minutes, or about 8 minutes. In some embodiments, the irradiation can be performed at a total light dose of between about one and about ten mW/mm², for example, at about 2 mW/mm², about 4 mW/mm², about 6 mW/mm², or about 8 mW/mm². In some embodiments, the irradiation can be performed at a total light dose of between about one and about ten mW/mm² and for a duration of between about 1 minute and about 10 minutes.

### B. Polymers

In some embodiments, a method disclosed herein comprises irradiating a first polynucleotide immobilized on a substrate with a first light while a second polynucleotide immobilized on the substrate is not irradiated with the first light, wherein the first polynucleotide is bound to a first photo-cleavable polymer that inhibits or blocks hybridization and/or ligation to the first polynucleotide, and the second polynucleotide is bound to a second photo-cleavable polymer that inhibits or blocks hybridization and/or ligation to the second polynucleotide, thereby cleaving the first photo-cleavable polymer such that the inhibition or blocking of hybridization and/or ligation to the first polynucleotide is reduced or eliminated, whereas hybridization and/or ligation to the second polynucleotide remains inhibited or blocked by the second photo-cleavable polymer, wherein a first barcode is attached to the first polynucleotide via hybridization and/or ligation, thereby providing on the substrate an array comprising the first and second polynucleotides, wherein the first polynucleotide is barcoded with the first barcode and the second polynucleotide is not barcoded with the first barcode.

In some embodiments, the method further comprises irradiating the second polynucleotide with a second light, thereby cleaving the second photo-cleavable polymer such that the inhibition or blocking of hybridization and/or ligation to the second polynucleotide is reduced or eliminated. In some embodiments, the second polynucleotide is irradiated with the second light while the first polynucleotide is not irradiated with the second light. In some embodiments, the method further comprises attaching a second barcode to the second polynucleotide via hybridization and/or ligation, thereby providing on the substrate an array comprising the first polynucleotide barcoded with the first barcode and the second polynucleotide barcoded with the second barcode. In some embodiments, hybridization and/or ligation to the first polynucleotide barcoded with the first barcode is inhibited or blocked. In some embodiments, the first barcode comprises a first photo-cleavable moiety that inhibits or blocks hybridization and/or ligation, thereby inhibiting or blocking hybridization and/or ligation to the first polynucleotide barcoded with the first barcode. In some embodiment, the first photo-cleavable moiety comprises a photo-caged nucleobase, a photo-cleavable linker, a photo-cleavable hairpin and/or a photo-caged 3'-hydroxyl group. In some embodiments, the first barcode is a DNA oligonucleotide. In some embodiments, the first barcode is between about 5 and about 20 nucleotides in length. In some embodiments, the substrate comprises a plurality of differentially barcoded polynucleotides immobilized thereon. In some embodiments, the irradiating comprises using a photomask to selectively irradiate the first polynucleotide or the second polynucleotide. In some embodiments, the attachment of the first barcode and/or the second barcode comprises ligating one end of the first/second barcode to one end of the first/second polynucleotide, respectively. In some embodiments, the attachment of the first barcode comprises hybridizing one end of the first barcode and one end of the first polynucleotide to a first splint. In some embodiments, the method further comprises ligating the first barcode to the first polynucleotide hybridized to the first splint. In some embodiments, the first barcode is directly ligated to the first polynucleotide, without gap filling. In some embodiments, ligating the first barcode to the first polynucleotide is preceded by gap filling. In some embodiments, the first splint is a DNA oligonucleotides at least 4 nucleotides in length. In some embodiments, the first photo-cleavable polymer and/or the second photo-cleavable polymer are UV degradable. In some embodiments, the first photo-cleavable polymer and/or the second photo-cleavable polymer comprise a polyethylenimine (PEI).

In some embodiments, a method disclosed herein comprises: (a1) irradiating polynucleotide P1 immobilized on a substrate with light while polynucleotide P2 immobilized on the substrate is photomasked, wherein polynucleotides P1 and P2 are bound to a photo-cleavable polymer that inhibits or blocks hybridization and/or ligation to P1 and P2, respectively, thereby cleaving the photo-cleavable polymer to allow hybridization and/or ligation to P1, whereas hybridization and/or ligation to P2 remain inhibited or blocked by the photo-cleavable polymer; and (b1) attaching barcode 1A to P1 via hybridization and/or ligation to form a barcoded polynucleotide 1A-P1, thereby providing on the substrate an array comprising polynucleotides 1A-P1 and P2. In some embodiments, the method further comprises: (c1) irradiating P2 with light, thereby cleaving the photo-cleavable polymer to allow hybridization and/or ligation to P2; and (d1) attaching barcode 1B to P2 via hybridization and/or ligation to form a barcoded polynucleotide 1B-P2, thereby providing on the substrate an array comprising barcoded polynucleotides 1A-P1 and 1B-P2. In some embodiments, polynucleotides of different nucleic acid sequences are immobilized on the substrate in a pattern comprising rows and columns prior to the irradiation.

In any of the preceding embodiments, the first polynucleotide irradiated with the first light and the second polynucleotide that is not irradiated with the first light can be in an overlapping area between the first and second areas for microfluidic channel-guided oligonucleotide delivery and attachments. For instance, the first and second polynucleotides can be generated using microfluidic devices and can be in an intersectional area as shown in **FIG. 2C****,** but the first polynucleotide is irradiated while the second polynucleotide is not irradiated such that a third oligonucleotide comprising a third barcode sequence (or a third part of a barcode sequence to be assembled) can be selectively attached to the first polynucleotide but not to the second polynucleotide.

In some embodiments, the oligonucleotide molecules are protected from hybridization by a polymer binding to the oligonucleotide molecules. In some embodiments, the oligonucleotide molecules are protected from ligation by a polymer binding to the oligonucleotide molecules. In some embodiments, the oligonucleotide molecules are protected from hybridization and ligation by a polymer binding to the oligonucleotide molecules. In some embodiments, the polymer is a photo-cleavable polymer. In some embodiments, the polymer (e.g., photo-cleavable polymer) binds to the oligonucleotide molecules in a non-sequence specific manner. In some embodiments, the photo-cleavable polymers in irradiated regions are cleaved and the photo-cleavable polymers in masked or non-irradiated regions are not cleaved.

In some embodiments, a photo-cleavable polymer disclosed herein is not part of an oligonucleotide. In some embodiments, a photo-cleavable polymer disclosed herein is not covalently bonded to an oligonucleotide. In some embodiments, a photo-cleavable polymer disclosed herein is noncovalently bound to an oligonucleotide. In some embodiments, the oligonucleotide is prevented from hybridization to a nucleic acid such as a splint. In some embodiments, a photo-cleavable polymer disclosed herein inhibits or blocks ligation to either end of the oligonucleotide, while hybridization of a nucleic acid to the oligonucleotide may or may not be inhibited or blocked. For example, the photo-cleavable polymer bound to an oligonucleotide may inhibit or block the 3' or 5' end of the oligonucleotide from chemical or enzymatic ligation, *e.g*., even when a splint may hybridize to the oligonucleotide in order to bring a ligation partner in proximity to the 3' or 5' end of the oligonucleotide. In some embodiments, the photo-cleavable polymer may cap the 3' or 5' end of the oligonucleotide.

In some embodiments, the photo-cleavable polymer is UV degradable. In some embodiments, the photo-cleavable polymer comprises a UV-degradable group (e.g., a UV-degradable functional moiety). In some embodiments, the UV-degradable group is within the backbone or at each subunit of the photo-cleavable polymer. In some embodiments, the UV-degradable group comprises a nitrobenzyl group, e.g., within a PEG (polyethylene glycol), a PDMS (polydimethylsiloxane), or a polyethylenimine (PEI), for example, in the polymer backbone or at each subunit. Complete cleavage of the nitrobenzyl group(s) is not required for nucleic acid release. In some embodiments, cleavage of a portion of the UV-degradable groups is sufficient to render the oligonucleotides available for hybridization and/or ligation.

In some embodiments, the photo-cleavable polymer is synthetic, semisynthetic, or natural. In some embodiments, the photo-cleavable polymer comprises a material selected from the group consisting of a PEG (polyethylene glycol), a PDMS (polydimethylsiloxane), a polyethylenimine (PEI), a polyacrylate, a lipid, a nanoparticle, a DNA, an RNA, a synthetic oligodeoxynucleotide (ODN), a xeno nucleic acid (XNA), a peptide nucleic acid (PNA), a locked nucleic acid (LNA), a 1,5-anhydrohexitol nucleic acid (HNA), a cyclohexene nucleic acid (CeNA), a threose nucleic acid (TNA), a glycol nucleic acid (GNA), a fluoro arabino nucleic acid (FANA), and a polypeptide. In some embodiments, the polyacrylate and/or the lipid is cationic, optionally wherein the cationic lipid is Lipofectamine. In some embodiments, the photo-cleavable polymer comprises the formula of (dNTP)₆-PC-(dNTP)₆-PC-(dNTP)₆-PC-(dNTP)₆, wherein PC is a photo-cleavable moiety.

### C. Oligonucleotides Comprising Photo-cleavable Moieties

In some embodiments, a method disclosed herein comprises irradiating a first polynucleotide immobilized on a substrate with a first light while a second polynucleotide immobilized on the substrate is not irradiated with the first light, wherein the first polynucleotide comprises a first photo-cleavable moiety that inhibits or blocks hybridization and/or ligation to the first polynucleotide, and the second polynucleotide comprises a second photo-cleavable moiety that inhibits or blocks hybridization and/or ligation to the second polynucleotide, thereby cleaving the first photo-cleavable moiety such that the inhibition or blocking of hybridization and/or ligation to the first polynucleotide is reduced or eliminated, whereas hybridization and/or ligation to the second polynucleotide remain inhibited or blocked by the second photo-cleavable moiety, wherein a first barcode is attached to the first polynucleotide via hybridization and/or ligation, thereby providing on the substrate an array comprising the first and second polynucleotides, wherein the first polynucleotide is barcoded with the first barcode and the second polynucleotide is not barcoded with the first barcode.

In some embodiments, the method further comprises irradiating the second polynucleotide with a second light, thereby cleaving the second photo-cleavable moiety such that the inhibition or blocking of hybridization and/or ligation to the second polynucleotide is reduced or eliminated. In some embodiments, the second polynucleotide is irradiated with the second light while the first polynucleotide is not irradiated with the second light. In some embodiments, the method further comprises attaching a second barcode to the second polynucleotide via hybridization and/or ligation, thereby providing on the substrate an array comprising the first polynucleotide barcoded with the first barcode and the second polynucleotide barcoded with the second barcode. In some embodiments, hybridization and/or ligation to the first polynucleotide barcoded with the first barcode is inhibited or blocked, and/or hybridization and/or ligation to the second polynucleotide barcoded with the second barcode is inhibited or blocked. In some embodiments, the first barcode comprises a third photo-cleavable moiety that inhibits or blocks hybridization and/or ligation, thereby inhibiting or blocking hybridization and/or ligation to the first polynucleotide barcoded with the first barcode. In some embodiments, the first photo-cleavable moiety and/or the second photo-cleavable moiety comprise a photo-caged nucleobase. In some embodiments, the first photo-cleavable moiety and/or the second photo-cleavable moiety comprise a photo-cleavable hairpin. In some embodiments, the first photo-cleavable moiety and/or the second photo-cleavable moiety comprise a photo-caged 3'-hydroxyl group. In some embodiments, the substrate comprises a plurality of differentially barcoded polynucleotides immobilized thereon. In some embodiments, irradiating the sample comprises using a photomask to selectively irradiate the first polynucleotide or the second polynucleotide. In some embodiments, the attachment of the first barcode-comprises ligating one end of the first barcode to one end of the first polynucleotide. In some embodiments, the attachment of the first barcode comprises hybridizing one end of the first barcode and one end of the first polynucleotide to a first splint. In some embodiments, the method further comprises ligating the first barcode to the first polynucleotide hybridized to the first splint. In some embodiments, ligating the first barcode to the first polynucleotide, respectively, is preceded by gap filling.

In some embodiments, a method disclosed herein comprises (a1) irradiating polynucleotide P1 immobilized on a substrate with light while polynucleotide P2 immobilized on the substrate is photomasked, wherein polynucleotides P1 and P2 comprise a photo-cleavable moiety that inhibits or blocks hybridization and/or ligation to P1 and P2, respectively, thereby cleaving the photo-cleavable moiety to allow hybridization and/or ligation to P1, whereas hybridization and/or ligation to P2 remain inhibited or blocked by the photo-cleavable moiety; and (b1) attaching barcode 1A to P1 via hybridization and/or ligation to form a barcoded polynucleotide 1A-P1, wherein barcode 1A comprises the photo-cleavable moiety which inhibits or blocks hybridization and/or ligation to 1A-P1, thereby providing on the substrate an array comprising polynucleotides 1A-P1 and P2 each comprising the photo-cleavable moiety that inhibits or blocks hybridization and/or ligation. In some embodiments, the method further comprises: (c1) irradiating P2 with light while 1A-P1 is photomasked, thereby cleaving the photo-cleavable moiety to allow hybridization and/or ligation to P2, whereas hybridization and/or ligation to 1A-P1 remain inhibited or blocked by the photo-cleavable moiety; and (d1) attaching barcode 1B to P2 via hybridization and/or ligation to form a barcoded polynucleotide 1B-P2, wherein barcode 1B comprises the photo-cleavable moiety which inhibits or blocks hybridization and/or ligation to 1B-P2, thereby providing on the substrate an array comprising barcoded polynucleotides 1A-P1 and 1B-P2 each comprising the photo-cleavable moiety that inhibits or blocks hybridization and/or ligation. In some embodiments, the method further comprises: (a2) irradiating one of 1A-P1 and 1B-P2 with light while the other is photomasked, thereby cleaving the photo-cleavable moiety to allow hybridization and/or ligation to the irradiated polynucleotide, whereas hybridization and/or ligation to the photomasked polynucleotide remains inhibited or blocked by the photo-cleavable moiety; and (b2) attaching barcode 2A to the irradiated polynucleotide via hybridization and/or ligation to form a 2A-barcoded polynucleotide, wherein barcode 2A comprises the photo-cleavable moiety which inhibits or blocks hybridization and/or ligation, thereby providing on the substrate an array comprising barcoded polynucleotides each comprising the photo-cleavable moiety that inhibits or blocks hybridization and/or ligation. In some embodiments, the method further comprises (c2) irradiating the photomasked polynucleotide in step a2 with light while the 2A-barcoded polynucleotide is photomasked, thereby cleaving the photo-cleavable moiety to allow hybridization and/or ligation, whereas hybridization and/or ligation to the 2A-barcoded polynucleotide remain inhibited or blocked by the photo-cleavable moiety; and (d2) attaching barcode 2B to the irradiated polynucleotide in step c2 via hybridization and/or ligation to form a 2B-barcoded polynucleotide, wherein barcode 2B comprises the photo-cleavable moiety which inhibits or blocks hybridization and/or ligation, thereby providing on the substrate an array comprising barcoded polynucleotides each comprising the photo-cleavable moiety that inhibits or blocks hybridization and/or ligation. In some embodiments, steps a1-d1 form round 1 and steps a2-d2 form round 2, the method further comprising steps a*i-*d*i* in round *i,* wherein barcodes *i*A and *i*B are attached to provide barcoded polynucleotides on the substrate, and wherein *i* is an integer greater than 2.

In some embodiments, the photo-cleavable moiety comprises a photo-caged nucleobase. In some embodiments, the photo-caged nucleobase is a photo-caged deoxythymidine (dT). In some embodiments, the photo-cleavable moiety comprises the following structure: 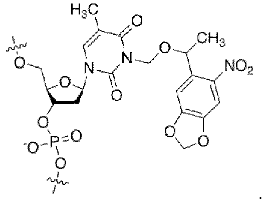 In some embodiments, the photo-cleavable moiety comprises a photo-cleavable hairpin. In some embodiments, the photo-cleavable moiety comprises the following structure: 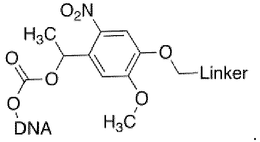 In some embodiments, the photo-cleavable moiety comprises a photo-caged 3'-hydroxyl group. In some embodiments, the photo-cleavable moiety comprises the following structure: 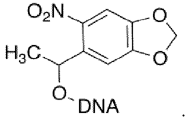 In some embodiments, polynucleotides of different nucleic acid sequences are immobilized on the substrate in a pattern comprising rows and columns prior to the irradiation.

In any of the preceding embodiments, the first polynucleotide irradiated with the first light and the second polynucleotide that is not irradiated with the first light can be in an overlapping area between the first and second areas for microfluidic channel-guided oligonucleotide delivery and attachments. For instance, the first and second polynucleotides can be generated using microfluidic devices and can be in an intersectional area as shown in **FIG. 2C****,** but the first polynucleotide is irradiated while the second polynucleotide is not irradiated such that a third oligonucleotide comprising a third barcode sequence (or a third part of a barcode sequence to be assembled) can be selectively attached to the first polynucleotide but not to the second polynucleotide.

In some embodiments, the oligonucleotide molecules are protected from hybridization by a protective group of each oligonucleotide molecule. In some embodiments, the oligonucleotide molecules are protected from ligation by a protective group of each oligonucleotide molecule. In some embodiments, the oligonucleotide molecules are protected from hybridization and ligation by a protective group of each oligonucleotide molecule. In some embodiments, the protective group is a photo-cleavable protective group. In some embodiments, the photo-cleavable protective groups in irradiated regions are cleaved and the photo-cleavable protective groups in masked or non-irradiated regions are not cleaved. Specifically, in some embodiments, the oligonucleotide molecules are protected from hybridization using photo-caged oligonucleotides. In some embodiments, the oligonucleotide molecules are protected from ligation using photo-caged oligonucleotides. In some embodiments, the oligonucleotide molecules are protected from hybridization and ligation using photo-caged oligonucleotides. In some embodiments, the photo-caged oligonucleotides comprises one or more photo-cleavable moieties, such as a photo-cleavable protective group. For example, hybridization can be blocked using a synthetic nucleotide with a photo-cleavable protecting group on a nucleobase and/or a photo-cleavable hairpin that dissociates upon cleavage. In other examples, ligation can be controlled using a photo-cleavable moiety, such as a photo-caged 3'-hydroxyl group.

In some embodiments, a photo-cleavable moiety disclosed herein is part of an oligonucleotide (e.g., a first oligonucleotide or a second oligonucleotide), such as oligonucleotide molecules in one or more other regions on the substrate, and inhibits or blocks hybridization to the oligonucleotide (e.g., the hybridization of a splint and/or a third oligonucleotide to the first and/or second oligonucleotide), but does not inhibit or block hybridization to the oligonucleotide molecules in one or more regions on the substrate. In some embodiments, the oligonucleotide is prevented from hybridization to a nucleic acid such as a splint. In some embodiments, a photo-cleavable moiety disclosed herein is part of an oligonucleotide and inhibits or blocks ligation to either end of the oligonucleotide, while hybridization of a nucleic acid to the oligonucleotide may or may not be inhibited or blocked. For example, the photo-cleavable moiety may inhibit or block the 3' or 5' end of the oligonucleotide from chemical or enzymatic ligation, *e.g.*, even when a splint may hybridize to the oligonucleotide in order to bring a ligation partner in proximity to the 3' or 5' end of the oligonucleotide. In some embodiments, the photo-cleavable moiety may cap the 3' or 5' end of the oligonucleotide.

In some embodiments, the irradiation results in cleavage of the photo-cleavable moiety such that the inhibition or blocking of hybridization and/or ligation to the oligonucleotide molecules in the one or more regions is reduced or eliminated, whereas hybridization and/or ligation to the oligonucleotide nucleotide molecules in one or more other regions remain inhibited or blocked by a second photo-cleavable moiety.

In any of the embodiments herein, physical masks, *e.g.,* a photolithography mask which is an opaque plate or film with transparent areas that allow light to shine through in a defined pattern, may be used. In any of the embodiments herein, different protection groups and/or photolabile groups may be used. In any of the embodiments herein, the light can have a wavelength between about 365 nm and about 440 nm, for example, about 366 nm, 405 nm, or 436 nm.

### IV. COMPOSITIONS, HITS, AND ARTICLES OF MANUFACTURE

Also provided are compositions produced according to the methods described herein. These compositions include nucleic acid molecules and complexes, such as hybridization complexes, and kits and articles of manufacture (such as arrays) comprising such molecules and complexes. In other aspect, provided herein is an array of oligonucleotides produced by the method of any of the embodiments herein.

In some embodiments, the arrays are arrays of nucleic acids, including oligonucleotides, polynucleotides, cDNAs, mRNAs, synthetic mimetics thereof, and the like. Where the arrays are arrays of nucleic acids, the nucleic acids may be covalently attached to the arrays at any point along the nucleic acid chain, but are generally attached at one of their termini, e.g. the 3' or 5' terminus.

Arrays can be used to measure large numbers of analytes or proxies thereof simultaneously. In some embodiments, oligonucleotides are used, at least in part, to create an array. For example, one or more copies of a single species of oligonucleotide (e.g., capture probe) can correspond to or be directly or indirectly attached to a given feature in the array. In some embodiments, a given feature in the array includes two or more species of oligonucleotides (e.g., capture probes). In some embodiments, the two or more species of oligonucleotides (e.g., capture probes) attached directly or indirectly to a given feature on the array include a common (e.g., identical) spatial barcode.

In some embodiments, an array can include a capture probe attached directly or indirectly to the substrate. The capture probe can include a capture domain (e.g., a nucleotide sequence) that can specifically bind (e.g., hybridize) to a target analyte (e.g., mRNA, DNA, or protein) or a proxy thereof (e.g., a ligation product obtained by ligation of a probe pair to a target nucleic acid sequence) within a sample. In some embodiments, the binding of the capture probe to the target (e.g., hybridization) can be detected and quantified by detection of a visual signal, e.g., a fluorophore, a heavy metal (e.g., silver ion), or chemiluminescent label, which has been incorporated into the target. In some embodiments, the intensity of the visual signal correlates with the relative abundance of each analyte in the biological sample. Since an array can contain thousands or millions of capture probes (or more), an array can interrogate many analytes or proxies thereof in parallel. In some embodiments, the binding (e.g., hybridization) of the capture probe to the target can be detected and quantified by creation of a molecule (e.g., cDNA from captured mRNA generated using reverse transcription) that is removed from the array, and sequenced.

Kits for use in analyte detection assays are provided. In some embodiments, the kit at least includes an array disclosed herein. The kits may further include one or more additional components necessary for carrying out an analyte detection assay, such as sample preparation reagents, buffers, labels, and the like. As such, the kits may include one or more containers such as vials or bottles, with each container containing a separate component for the assay, and reagents for carrying out an array assay such as a nucleic acid hybridization assay or the like. The kits may also include a denaturation reagent for denaturing the analyte, buffers such as hybridization buffers, wash mediums, enzyme substrates, reagents for generating a labeled target sample such as a labeled target nucleic acid sample, negative and positive controls and written instructions for using the subject array assay devices for carrying out an array based assay. The instructions may be printed on a substrate, such as paper or plastic, etc. As such, the instructions may be present in the kits as a package insert, in the labeling of the container of the kit or components thereof (*e.g.,* associated with the packaging or sub-packaging) *etc.*

In particular embodiments, provided herein are kits and compositions for spatial array-based analysis of biological samples. Array-based spatial analysis methods involve the transfer of one or more analytes or proxies thereof from a biological sample to an array of features on a substrate, where each feature is associated with a unique spatial location on the array. Subsequent analysis of the transferred analytes includes determining the identity of the analytes and the spatial location of each analyte within the biological sample. The spatial location of each analyte within the biological sample is determined based on the feature to which each analyte is bound on the array, and the feature's relative spatial location within the array. In some embodiments, the array of features on a substrate comprise a spatial barcode that corresponds to the feature's relative spatial location within the array. Each spatial barcode of a feature may further comprise a fluorophore, to create a fluorescent hybridization array. A feature may comprise UMIs that are generally unique per nucleic acid molecule in the feature - this is so the number of unique molecules can be estimated, as opposed to an artifact in experiments or PCR amplification bias that drives amplification of smaller, specific nucleic acid sequences.

In particular embodiments, the kits and compositions for spatial array-based analysis provide for the detection of differences in an analyte level (*e.g.,* gene and/or protein expression) within different cells in a tissue of a mammal or within a single cell from a mammal. For example, the kits and compositions can be used to detect the differences in analyte levels (*e.g.,* gene and/or protein expression) within different cells in histological slide samples (e.g., intact tissue section), the data from which can be reassembled to generate a three-dimensional map of analyte levels (*e.g.,* gene and/or protein expression) of a tissue sample obtained from a mammal, *e.g.,* with a degree of spatial resolution (*e.g.,* single-cell scale resolution).

Also provided herein are arrays comprising any one or more of the molecules, complexes, and/or compositions disclosed herein. Typically, an array includes at least two distinct nucleic acids that differ by monomeric sequence immobilized on, *e.g*., covalently to, different and known locations on the substrate surface. In certain embodiments, each distinct nucleic acid sequence of the array is typically present as a composition of multiple copies of the polymer on the substrate surface, *e.g.* as a spot on the surface of the substrate. The number of distinct nucleic acid sequences, and hence spots or similar structures, present on the array may vary, but is generally at least, usually at least 5 and more usually at least 10, where the number of different spots on the array may be as a high as 50, 100, 500, 1000, 10,000 1,000,000, 10,000,000 or higher, depending on the intended use of the array. The spots of distinct polymers present on the array surface are generally present as a pattern, where the pattern may be in the form of organized rows and columns of spots, *e.g.* a grid of spots, across the substrate surface, a series of curvilinear rows across the substrate surface, *e.g.* a series of concentric circles or semicircles of spots, and the like. The density of spots present on the array surface may vary, but is generally at least about 10 and usually at least about 100 spots/cm ², where the density may be as high as 10⁶ or higher, or about 10⁵ spots/cm². In other embodiments, the polymeric sequences are not arranged in the form of distinct spots, but may be positioned on the surface such that there is substantially no space separating one polymer sequence/feature from another. The density of nucleic acids within an individual feature on the array may be as high as 1,000, 10,000, 25,000, 50,000, 100,000, 500,000, 1,000,000, or higher per square micron depending on the intended use of the array.

In some embodiments, the arrays are arrays of nucleic acids, including oligonucleotides, polynucleotides, cDNAs, mRNAs, synthetic mimetics thereof, and the like. Where the arrays are arrays of nucleic acids, the nucleic acids may be covalently attached to the arrays at any point along the nucleic acid chain, but are generally attached at one of their termini, e.g. the 3' or 5' terminus.

Arrays can be used to measure large numbers of analytes or proxies thereof simultaneously. In some embodiments, oligonucleotides are used, at least in part, to create an array. For example, one or more copies of a single species of oligonucleotide (e.g., capture probe) can correspond to or be directly or indirectly attached to a given feature in the array. In some embodiments, a given feature in the array includes two or more species of oligonucleotides (e.g., capture probes). In some embodiments, the two or more species of oligonucleotides (e.g., capture probes) are attached directly or indirectly to a given feature on the array include a common (e.g., identical) spatial barcode.

In some embodiments, an array can include a capture probe attached directly or indirectly to the substrate. The capture probe can include a capture domain (e.g., a nucleotide or amino acid sequence) that can specifically bind (e.g., hybridize) to a target analyte (e.g., mRNA, DNA, or protein) within a sample. In some embodiments, the binding of the capture probe to the target (e.g., hybridization) can be detected and quantified by detection of a visual signal, e.g., a fluorophore, a heavy metal (e.g., silver ion), or chemiluminescent label, which has been incorporated into the target. In some embodiments, the intensity of the visual signal correlates with the relative abundance of each analyte in the biological sample. Since an array can contain thousands or millions of capture probes (or more), an array can interrogate many analytes in parallel. In some embodiments, the binding (e.g., hybridization) of the capture probe to the target can be detected and quantified by creation of a molecule (e.g., cDNA from captured mRNA generated using reverse transcription) that is removed from the array, and sequenced.

The subject arrays find use in a variety of different applications, where such applications are generally analyte detection applications in which the presence of a particular analyte in a given sample is detected at least qualitatively, if not quantitatively. Protocols for carrying out such assays are well known to those of skill in the art and need not be described in great detail here. Generally, the sample suspected of comprising the analyte of interest or proxy thereof is contacted with an array produced according to the subject methods under conditions sufficient for the analyte or proxy thereof to bind to its respective binding pair member that is present on the array. Thus, if the analyte of interest is present in the sample, it binds to the array at the site of its complementary binding member and a complex is formed on the array surface. The presence of this binding complex on the array surface is then detected, e.g. through use of a signal production system, e.g. an isotopic or fluorescent label present on the analyte, e.g., through sequencing the analyte or product thereof, etc. The presence of the analyte in the sample is then deduced from the detection of binding complexes on the substrate surface, or sequence detection and/or analysis (e.g., by sequencing) on molecules indicative of the formation of the binding complex. In some embodiments, RNA molecules (e.g., mRNA) from a sample are captured by oligonucleotides (e.g., probes comprising a barcode and a poly(dT) sequence) on an array prepared by a method disclosed herein, cDNA molecules are generated via reverse transcription of the captured RNA molecules, and the cDNA molecules (e.g., a first strand cDNA) or portions or products (e.g., a second strand cDNA synthesized using a template switching oligonucleotide) thereof can be separated from the array and sequenced. Sequencing data obtained from molecules prepared on the array can be used to deduce the presence/absence or an amount of the RNA molecules in the sample.

Specific analyte detection applications of interest include hybridization assays in which the nucleic acid arrays of the present disclosure are employed. In these assays, a sample of target nucleic acids or a sample comprising intact cells or a tissue section is first prepared, where preparation may include labeling of the target nucleic acids with a label, e.g. a member of signal producing system. Following sample preparation, the sample is contacted with the array under hybridization conditions, whereby complexes are formed between target nucleic acids that are complementary to probe sequences attached to the array surface. The formation and/or presence of hybridized complexes is then detected, e.g., by analyzing molecules that are generated following the formation of the hybridized complexes, such as cDNA or a second strand generated from an RNA captured on the array. Specific hybridization assays of interest which may be practiced using the subject arrays include: gene discovery assays, differential gene expression analysis assays; nucleic acid sequencing assays, single nucleotide polymorphism assays, copy number variation assays, and the like.

### SPATIAL ANALYSIS

In some aspects, provided herein is a method for construction of a hybridization complex or an array comprising nucleic acid molecules and complexes. Oligonucleotide probes for capturing analytes or proxies thereof may be generated using a method disclosed herein, for example, using two, three, four, or more rounds of hybridization and ligation as shown in **FIG. 6****.**

In some embodiments, the oligonucleotide probe for capturing analytes or proxies thereof may be generated from an existing array with a ligation strategy. In some embodiments, an array containing a plurality of oligonucleotides (*e.g., in situ* synthesized oligonucleotides) can be modified to generate a variety of oligonucleotide probes. The oligonucleotides can include various domains such as, spatial barcodes, UMIs, functional domains (*e.g*., sequencing handle), cleavage domains, and/or ligation handles.

In some embodiments, an oligonucleotide probe can directly capture an analyte, such as mRNAs based on a poly(dT) capture domain on the oligonucleotide probe immobilized on an array. In some embodiments, the oligonucleotide probe is used for indirect analyte capture. For example, in fixed samples, such as FFPE, a probe pair can be used, and probes pairs can be target specific for each gene of the transcriptome. The probe pairs are delivered to a tissue section (which is itself on a spatial array) with a decrosslinking agent and a ligase, and the probe pairs are left to hybridize and ligate, thereby forming ligation products. The ligation products contain sequences in one or more overhangs of the probes, and the overhangs are not target specific and are complementary to capture domains on oligonucleotides immobilized on a spatial array, thus allowing the ligation product (which is a proxy for the analyte) to be captured on the array, processed, and subsequently analyzed (e.g., using a sequencing method).

A "spatial barcode" may comprise a contiguous nucleic acid segment or two or more non-contiguous nucleic acid segments that function as a label or identifier that conveys or is capable of conveying spatial information. In some embodiments, a capture probe includes a spatial barcode that possesses a spatial aspect, where the barcode is associated with a particular location within an array or a particular location on a substrate. A spatial barcode can be part of a capture probe on an array generated herein. A spatial barcode can also be a tag attached to an analyte (e.g., a nucleic acid molecule) or a combination of a tag in addition to an endogenous characteristic of the analyte (e.g., size of the analyte or end sequence(s)). A spatial barcode can be unique. In some embodiments where the spatial barcode is unique, the spatial barcode functions both as a spatial barcode and as a unique molecular identifier (UMI), associated with one particular capture probe. Spatial barcodes can have a variety of different formats. For example, spatial barcodes can include polynucleotide spatial barcodes; random nucleic acid and/or amino acid sequences; and synthetic nucleic acid and/or amino acid sequences. In some embodiments, a spatial barcode is attached to an analyte in a reversible or irreversible manner. In some embodiments, a spatial barcode is added to, for example, a fragment of a DNA or RNA sample before sequencing of the sample. In some embodiments, a spatial barcode allows for identification and/or quantification of individual sequencing-reads. In some embodiments, a spatial barcode is a used as a fluorescent barcode for which fluorescently labeled oligonucleotide probes hybridize to the spatial barcode.

In some embodiments, a spatial array is generated after ligating capture domains (*e.g.,* poly(T) or gene specific capture domains) to the oligonucleotide molecule (*e.g.,* generating capture oligonucleotides). The spatial array can be used with any of the spatial analysis methods described herein. For example, a biological sample (e.g., a tissue section) can be provided to the generated spatial array. In some embodiments, the biological sample is permeabilized. In some embodiments, the biological sample is permeabilized under conditions sufficient to allow one or more analytes (or proxies thereof) present in the biological sample to interact with the capture probes of the spatial array. After capture of analytes from the biological sample, the analytes can be analyzed (*e.g*., reverse transcribed, amplified, and/or sequenced) by any of the variety of methods described herein.

Sequential hybridization/ligation of various domains can be used to generate an oligonucleotide probe for capturing analytes or proxies thereof, by a photo-hybridization/ligation method described herein. For example, an oligonucleotide can be immobilized on a substrate (e.g., an array) and may comprise a functional sequence such as a primer sequence. In some embodiments, the primer sequence is a sequencing handle that comprises a primer binding site for subsequent processing. The primer sequence can generally be selected for compatibility with any of a variety of different sequencing systems, e.g., 454 Sequencing, Ion Torrent Proton or PGM, Illumina X10, PacBio, Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Roche 454 sequencing, Ion Torrent Proton or PGM sequencing, Illumina X10 sequencing, PacBio SMRT sequencing, and Oxford Nanopore sequencing. Further, in some embodiments, functional sequences can be selected for compatibility with other sequencing systems, including non-commercialized sequencing systems.

In some embodiments, in a first round hybridization/ligation (using microfluidic channel delivery or photolithography), an oligonucleotide comprising a part of a barcode (*e.g*., part A of the barcode) is attached to the oligonucleotide molecule comprising the primer (e.g., R1 primer). In some embodiments, the barcode part can be common to all of the oligonucleotide molecules in a given feature. In some embodiments, the barcode part can be common to all of the oligonucleotide molecules in multiple substrate regions (e.g., features) in the same cycle. In some embodiments, the barcode part can be different for oligonucleotide molecules in different substrate regions (e.g., features) in different cycles. In some embodiments, a splint with a sequence complementary to a portion of the primer of the immobilized oligonucleotide and an additional sequence complementary to a portion of the oligonucleotide comprising the part of the barcode (e.g., part A of the barcode) facilitates the ligation of the immobilized oligonucleotide and the oligonucleotide comprising the barcode part. In some embodiments, the splint for attaching the part of the barcode of various sequences to different substrate regions (e.g., features) is common among the cycles of the same round. In some embodiments, the splint for attaching the part of the barcode of various sequences to different substrate regions (e.g., features) can be different among the cycles of the same round. In some embodiments, the splint for attaching the part of the barcode may comprise a sequence complementary to the part or a portion thereof.

A second round hybridization/ligation (using microfluidic channel delivery or photolithography) can involve the addition of another oligonucleotide comprising another part of a barcode (*e.g*., part B of the barcode) to the immobilized oligonucleotide molecule comprising the primer and part A of the barcode. As shown in **FIG. 6****,** in some embodiments, a splint with a sequence complementary to a portion of the immobilized oligonucleotide comprising part A of the barcode and an additional sequence complementary to a portion of the oligonucleotide comprising part B of the barcode facilitates the ligation of the oligonucleotide comprising part B and the immobilized oligonucleotide comprising part A. In some embodiments, the splint for attaching part B of various sequences to different substrate regions (e.g., features) is common among the cycles of the same round. In some embodiments, the splint for attaching part B to different substrate regions (e.g., features) can be different among the cycles of the same round. In some embodiments, the splint for attaching part B may comprise a sequence complementary to part B or a portion thereof and/or a sequence complementary to part A or a portion thereof.

A third round hybridization/ligation (using microfluidic channel delivery or photolithography) can involve the addition of another oligonucleotide comprising another part of a barcode (*e.g*., part C of the barcode), added to the immobilized oligonucleotide molecule comprising the primer, part A, and part B. In some embodiments, a splint with a sequence complementary to a portion of the immobilized oligonucleotide molecule comprising part B and an additional sequence complementary to a portion of the oligonucleotide comprising part C facilitates the ligation of the immobilized oligonucleotide molecule comprising part B and the oligonucleotide comprising part C. In some embodiments, the splint for attaching part C of various sequences to different substrate regions (e.g., features) is common among the cycles of the same round. In some embodiments, the splint for attaching part C to different substrate regions (e.g., features) can be different among the cycles of the same round. In some embodiments, the splint for attaching part C may comprise a sequence complementary to part C or a portion thereof and/or a sequence complementary to part B or a portion thereof.

A fourth round hybridization/ligation (using microfluidic channel delivery or photolithography) may be performed, which involves the addition of another oligonucleotide comprising another part of a barcode (*e.g*., part D of the barcode), added to the immobilized oligonucleotide molecule comprising the primer, part A, part B, and part C. In some embodiments, a splint with a sequence complementary to a portion of the immobilized oligonucleotide molecule comprising part C and an additional sequence complementary to a portion of the oligonucleotide comprising part D facilitates the ligation. In some embodiments, the splint for attaching part D of various sequences to different substrate regions (e.g., features) is common among the cycles of the same round. In some embodiments, the splint for attaching part D to different substrate regions (e.g., features) can be different among the cycles of the same round. In some embodiments, the splint for attaching part D may comprise a sequence complementary to part D or a portion thereof and/or a sequence complementary to part C or a portion thereof. In some embodiments, an oligonucleotide comprising part D further comprises a UMI and/or a capture domain.

In particular embodiments, provided herein are kits and compositions for spatial array-based analysis of biological samples. Array-based spatial analysis methods involve the transfer of one or more analytes or proxies thereof from a biological sample to an array of features on a substrate, where each feature is associated with a unique spatial location on the array. Subsequent analysis of the transferred analytes includes determining the identity of the analytes and the spatial location of each analyte within the biological sample. The spatial location of each analyte within the biological sample is determined based on the feature to which each analyte is bound on the array, and the feature's relative spatial location within the array. In some embodiments, the array of features on a substrate comprises a spatial barcode that corresponds to the feature's relative spatial location within the array. Each spatial barcode of a feature may further comprise a fluorophore, to create a fluorescent hybridization array. A feature may comprise UMIs that are generally unique per nucleic acid molecule in the feature so the number of unique molecules can be estimated, as opposed to an artifact in experiments or PCR amplification bias that drives amplification of smaller, specific nucleic acid sequences.

In particular embodiments, the kits and compositions for spatial array-based analysis provide for the detection of differences in an analyte level (*e.g.,* gene and/or protein expression) within different cells in a tissue of a mammal or within a single cell from a mammal. For example, the kits and compositions can be used to detect the differences in analyte levels (*e.g.,* gene and/or protein expression) within different cells in histological slide samples (e.g., tissue section), the data from which can be reassembled to generate a three-dimensional map of analyte levels (*e.g.,* gene and/or protein expression) of a tissue sample obtained from a mammal, *e.g.,* with a degree of spatial resolution (*e.g.,* single-cell resolution).

In some embodiments, an array generated using a method disclosed herein can be used in array-based spatial analysis methods which involve the transfer of one or more analytes from a biological sample to an array of features on a substrate, each of which is associated with a unique spatial location on the array. Subsequent analysis of the transferred analytes includes determining the identity of the analytes and the spatial location of each analyte within the sample. The spatial location of each analyte within the sample is determined based on the feature to which each analyte is bound in the array, and the feature's relative spatial location within the array.

There are at least two general methods to associate a spatial barcode with one or more neighboring cells, such that the spatial barcode identifies the one or more cells, and/or contents of the one or more cells, as associated with a particular spatial location. One general method is to drive target analytes out of a cell and towards the spatially-barcoded array. In some embodiments, the spatially-barcoded array populated with capture probes is contacted with a sample (e.g., a tissue section or a population of single cells), and the sample is permeabilized, allowing the target analyte to migrate away from the sample and toward the array. The target analyte interacts with a capture probe on the spatially-barcoded array. Once the target analyte hybridizes/is bound to the capture probe, the sample is optionally removed from the array and the capture probes are analyzed in order to obtain spatially-resolved analyte information. Methods for performing such spatial analysis of tissue sections are known in the art and include but are not limited to those methods disclosed in US Patent 10,030,261, US Patent 11,332,790 and US Patent Pub No. 20220127672 and US Patent Pub No. 20220106632.

Another general method is to cleave the spatially-barcoded capture probes from an array, and drive the spatially-barcoded capture probes towards and/or into or onto the sample. In some embodiments, the spatially-barcoded array populated with capture probes is contacted with a sample. The spatially-barcoded capture probes are cleaved and then interact with cells within the provided sample (See, for example, US Patent 11,352,659). The interaction can be a covalent or non-covalent cell-surface interaction. The interaction can be an intracellular interaction facilitated by a delivery system or a cell penetration peptide. Once the spatially-barcoded capture probe is associated with a particular cell, the sample can be optionally removed for analysis. The sample can be optionally dissociated before analysis. Once the tagged cell is associated with the spatially-barcoded capture probe, the capture probes can be analyzed (e.g., by sequencing) to obtain spatially-resolved information about the tagged cell.

Sample preparation may include placing the sample on a slide, fixing the sample, and/or staining the sample for imaging. The stained sample may be imaged on the array using both brightfield (to image the sample hematoxylin and eosin stain) and/or fluorescence (to image features) modalities. In some embodiments, target analytes are then released from the sample and capture probes forming the spatially-barcoded array hybridize or bind the released target analytes. The sample is then removed from the array and the capture probes cleaved from the array. The sample and array are then optionally imaged a second time in one or both modalities (brightfield and fluorescence) while the analytes are reverse transcribed into cDNA, and an amplicon library is prepared and sequenced. In some embodiments, the two sets of images can then be spatially-overlaid in order to correlate spatially-identified sample information. When the sample and array are not imaged a second time, a spot coordinate file may be supplied. The spot coordinate file can replace the second imaging step. Further, amplicon library preparation can be performed with a unique PCR adapter and sequenced.

In some embodiments, a spatially-labelled array on a substrate is used, where capture probes labelled with spatial barcodes are clustered at areas called features. The spatially-labelled capture probes can include a cleavage domain, one or more functional sequences, a spatial barcode, a unique molecular identifier, and a capture domain. The spatially-labelled capture probes can also include a 5' end modification for reversible attachment to the substrate. The spatially-barcoded array is contacted with a sample, and the sample is permeabilized through application of permeabilization reagents. Permeabilization reagents may be administered by placing the array/sample assembly within a bulk solution. Alternatively, permeabilization reagents may be administered to the sample via a diffusion-resistant medium and/or a physical barrier such as a lid, wherein the sample is sandwiched between the diffusion-resistant medium and/or barrier and the array-containing substrate. The analytes are migrated toward the spatially-barcoded capture array using any number of techniques disclosed herein. For example, analyte migration can occur using a diffusion-resistant medium lid and passive migration. As another example, analyte migration can be active migration, using an electrophoretic transfer system, for example. Once the analytes are in close proximity to the spatially-barcoded capture probes, the capture probes can hybridize or otherwise bind a target analyte. The sample can be optionally removed from the array.

Adapters and assay primers can be used to allow the capture probe or the analyte capture agent to be attached to any suitable assay primers and used in any suitable assays. A capture probe that includes a spatial barcode can be attached to a bead that includes a poly(dT) sequence. A capture probe including a spatial barcode and a poly(T) sequence can be used to assay multiple biological analytes as generally described herein (e.g., the biological analyte includes a poly(A) sequence or is coupled to or otherwise is associated with an analyte capture agent comprising a poly(A) sequence as the analyte capture sequence).

The capture probes can be optionally cleaved from the array, and the captured analytes can be spatially-tagged by performing a reverse transcriptase first strand cDNA reaction. A first strand cDNA reaction can be optionally performed using template switching oligonucleotides. For example, a template switching oligonucleotide can hybridize to a poly(C) tail added to a 3'end of the cDNA by a reverse transcriptase enzyme. The original mRNA template and template switching oligonucleotide can then be denatured from the cDNA and the barcoded capture probe can then hybridize with the cDNA and a complement of the cDNA can be generated. The first strand cDNA can then be purified and collected for downstream amplification steps. The first strand cDNA can be amplified using PCR, wherein forward and reverse primers flank the spatial barcode and target analyte regions of interest, generating a library associated with a particular spatial barcode. In some embodiments, the cDNA comprises a sequencing by synthesis (SBS) primer sequence. The library amplicons are sequenced and analyzed to decode spatial information.

In some embodiments, the sample is removed from the spatially-barcoded array and the spatially-barcoded capture probes are removed from the array for barcoded analyte amplification and library preparation. In some embodiments, the sample is removed from the spatially-barcoded array prior to removal of the spatially-barcoded capture probes from the array. Another embodiment includes performing first strand synthesis using template switching oligonucleotides on the spatially-barcoded array without cleaving the capture probes. Once the capture probes capture the target analyte(s), first strand cDNA created by template switching and reverse transcriptase is then denatured and the second strand is then extended. The second strand cDNA is then denatured from the first strand cDNA, neutralized, and transferred to a tube. cDNA quantification and amplification can be performed using standard techniques discussed herein. The cDNA can then be subjected to library preparation and indexing, including fragmentation, end-repair, A-tailing, and indexing PCR steps, and then sequenced.

### V. APPLICATIONS OF SPATIAL ARRAYS

The subject arrays find use in a variety of different applications, where such applications are generally analyte detection applications in which the presence of a particular analyte in a given sample is detected at least qualitatively, if not quantitatively. Protocols for carrying out such assays are well known to those of skill in the art and need not be described in great detail here. Generally, the sample suspected of comprising the analyte of interest (or proxies thereof) is contacted with an array produced according to the subject methods under conditions sufficient for the analyte to bind to its respective binding pair member that is present on the array. Thus, if the analyte of interest or proxy thereof is present in the sample, it binds to the array at the site of its complementary binding member and a complex is formed on the array surface. The presence of this binding complex on the array surface is then detected, e.g. through use of a signal production system, e.g. an isotopic or fluorescent label present on the analyte, etc., and/or through sequencing of one or more components of the binding complex or a product thereof. The presence of the analyte in the sample is then deduced from the detection of binding complexes on the substrate surface, or sequence detection and/or analysis (e.g., by sequencing) on molecules indicative of the formation of the binding complex. In some embodiments, RNA molecules (e.g., mRNA) from a sample are captured by oligonucleotides (e.g., probes comprising a barcode and a poly(dT) sequence) on an array prepared by a method disclosed herein, cDNA molecules are generated via reverse transcription of the captured RNA molecules, and the cDNA molecules (e.g., a first strand cDNA) or portions or products (e.g., a second strand cDNA synthesized using a template switching oligonucleotide) thereof can be separated from the array and sequenced. Sequencing data obtained from molecules prepared on the array can be used to deduce the presence/absence or an amount of the RNA molecules in the sample.

Specific analyte detection applications of interest include hybridization assays in which the nucleic acid arrays of the subject invention are employed. In these assays, a sample of target nucleic acids or a tissue section is first prepared, where preparation may include labeling of the target nucleic acids with a label, e.g. a member of signal producing system. Following sample preparation, the sample is contacted with the array under hybridization conditions, whereby complexes are formed between target nucleic acids that are complementary to probe sequences attached to the array surface. The formation and/or presence of hybridized complexes is then detected, e.g., by analyzing molecules that are generated following the formation of the hybridized complexes, such as cDNA or a second strand generated from an RNA captured on the array. Specific hybridization assays of interest which may be practiced using the subject arrays include: gene discovery assays, differential gene expression analysis assays; nucleic acid sequencing assays, and the like.

In some embodiments, an array generated using a method disclosed herein can be used in array-based spatial analysis methods which involve the transfer of one or more analytes from a biological sample to an array of features on a substrate, each of which is associated with a unique spatial location on the array. Subsequent analysis of the transferred analytes includes determining the identity of the analytes and the spatial location of each analyte within the sample. The spatial location of each analyte within the sample is determined based on the feature to which each analyte is bound in the array, and the feature's relative spatial location within the array.

### VI. TERMINOLOGY

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

As used in this specification and the appended claims, the singular forms "a," "an" and "the" include plural referents unless the context clearly dictates otherwise. Thus, for example, reference to "a molecule" includes a plurality of such molecules, and the like.

The term "about" as used herein refers to the usual error range for the respective value readily known to the skilled person in this technical field. Reference to "about" a value or parameter herein comprises (and describes) embodiments that are directed to that value or parameter *per se.*

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be comprised in the smaller ranges, and are also encompassed within the claimed subject matter, subject to any specifically excluded limit in the stated range. Where the stated range comprises one or both of the limits, ranges excluding either or both of those comprised limits are also comprised in the claimed subject matter. This applies regardless of the breadth of the range.

A sample such as a biological sample can include any number of macromolecules, for example, cellular macromolecules and organelles (e.g., mitochondria and nuclei). The biological sample can be a nucleic acid sample and/or protein sample. The biological sample can be a carbohydrate sample or a lipid sample. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions. In some embodiments, the biological sample may comprise cells which are deposited on a surface.

The term "barcode," comprises a label, or identifier, that conveys or is capable of conveying information (*e.g.,* information about an analyte in a sample, a bead, and/or a capture probe). A barcode can be part of an analyte, or independent of an analyte. A barcode can be attached to an analyte. A particular barcode can be unique relative to other barcodes. Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (*e.g.,* a barcode can be or can include a unique molecular identifier or "UMI").

Barcodes can spatially-resolve molecular components found in biological samples, for example, at single-cell scale resolution (*e.g.,* a barcode can be or can include a "spatial barcode"). In some embodiments, a barcode includes both a UMI and a spatial barcode. In some embodiments, a barcode includes two or more sub-barcodes that together function as a single barcode. For example, a polynucleotide barcode can include two or more polynucleotide sequences (*e.g.,* sub-barcodes) that are separated by one or more non-barcode sequences.

As used herein, the term "substrate" generally refers to a substance, structure, surface, material, means, or composition, which comprises a nonbiological, synthetic, nonliving, planar, spherical or flat surface. The substrate may include, for example and without limitation, semiconductors, synthetic metals, synthetic semiconductors, insulators and dopants; metals, alloys, elements, compounds and minerals; synthetic, cleaved, etched, lithographed, printed, machined and microfabricated slides, wafers, devices, structures and surfaces; industrial polymers, plastics, membranes; silicon, silicates, glass, metals and ceramics; wood, paper, cardboard, cotton, wool, cloth, woven and nonwoven fibers, materials and fabrics; nanostructures and microstructures. The substrate may comprise an immobilization matrix such as but not limited to, insolubilized substance, solid phase, surface, layer, coating, woven or nonwoven fiber, matrix, crystal, membrane, insoluble polymer, plastic, glass, biological or biocompatible or bioerodible or biodegradable polymer or matrix, microparticle or nanoparticle. Other examples may include, for example and without limitation, monolayers, bilayers, commercial membranes, resins, matrices, fibers, separation media, chromatography supports, polymers, plastics, glass, mica, gold, beads, microspheres, nanospheres, silicon, gallium arsenide, organic and inorganic metals, semiconductors, insulators, microstructures and nanostructures. Microstructures and nanostructures may include, without limitation, microminiaturized, nanometer-scale and supramolecular probes, tips, bars, pegs, plugs, rods, sleeves, wires, filaments, and tubes.

As used herein, the term "nucleic acid" generally refers to a polymer comprising one or more nucleic acid subunits or nucleotides. A nucleic acid may include one or more subunits selected from adenosine (A), cytosine (C), guanine (G), thymine (T) and uracil (U), or variants thereof. A nucleotide can include A, C, G, T or U, or variants thereof. A nucleotide can include any subunit that can be incorporated into a growing nucleic acid strand. Such subunit can be an A, C, G, T, or U, or any other subunit that is specific to one or more complementary A, C, G, T or U, or complementary to a purine (i.e., A or G, or variant thereof) or a pyrimidine (i.e., C, T or U, or variant thereof). A subunit can enable individual nucleic acid bases or groups of bases (e.g., AA, TA, AT, GC, CG, CT, TC, GT, TG, AC, CA, or uracil-counterparts thereof) to be resolved. In some examples, a nucleic acid is deoxyribonucleic acid (DNA) or ribonucleic acid (RNA), or derivatives thereof. A nucleic acid may be single-stranded or double-stranded.

The term "nucleic acid sequence" or "nucleotide sequence" as used herein generally refers to nucleic acid molecules with a given sequence of nucleotides, of which it may be desired to know the presence or amount. The nucleotide sequence can comprise ribonucleic acid (RNA) or DNA, or a sequence derived from RNA or DNA. Examples of nucleotide sequences are sequences corresponding to natural or synthetic RNA or DNA including genomic DNA and messenger RNA. The length of the sequence can be any length that can be amplified into nucleic acid amplification products, or amplicons, for example, up to about 20, 50, 100, 200, 300, 400, 500, 600, 700, 800, 1000, 1200, 1500, 2000, 5000, 10000 or more than 10000 nucleotides in length, or at least about 20, 50, 100, 200, 300, 400, 500, 600, 700, 800, 1000, 1200, 1500, 2000, 5000, 10000 nucleotides in length.

The terms "oligonucleotide" and "polynucleotide" are used interchangeably to refer to a single-stranded multimer of nucleotides from about 2 to about 500 nucleotides in length. Oligonucleotides can be synthetic, made enzymatically (e.g., via polymerization), or using a "split-pool" method. Oligonucleotides can include ribonucleotide monomers (i.e., can be oligoribonucleotides) and/or deoxyribonucleotide monomers (i.e., oligodeoxyribonucleotides). In some examples, oligonucleotides can include a combination of both deoxyribonucleotide monomers and ribonucleotide monomers in the oligonucleotide (e.g., random or ordered combination of deoxyribonucleotide monomers and ribonucleotide monomers). An oligonucleotide can be 4 to 10, 10 to 20, 21 to 30, 31 to 40, 41 to 50, 51 to 60, 61 to 70, 71 to 80, 80 to 100, 100 to 150, 150 to 200, 200 to 250, 250 to 300, 300 to 350, 350 to 400, or 400-500 nucleotides in length, for example. Oligonucleotides can include one or more functional moieties that are attached (e.g., covalently or non-covalently) to the multimer structure. For example, an oligonucleotide can include one or more detectable labels (e.g., a radioisotope or fluorophore).

As used herein, the term "adjacent" or "adjacent to," includes "next to," "adjoining," and "abutting." In one example, a first location is adjacent to a second location when the first location is in direct contact and shares a common border with the second location and there is no space between the two locations. In some cases, the adjacent is not diagonally adj acent.

An "adaptor," an "adapter," and a "tag" are terms that are used interchangeably in this disclosure, and refer to species that can be coupled to a polynucleotide sequence (in a process referred to as "tagging") using any one of many different techniques including (but not limited to) ligation, hybridization, and tagmentation. Adaptors can also be nucleic acid sequences that add a function, e.g., spacer sequences, primer sequences/sites, barcode sequences, unique molecular identifier sequences.

The terms "hybridizing," "hybridize," "annealing," and "anneal" are used interchangeably in this disclosure, and refer to the pairing of substantially complementary or complementary nucleic acid sequences within two different molecules. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

A "proximity ligation" is a method of ligating two (or more) nucleic acid sequences that are in proximity with each other through enzymatic means (e.g., a ligase). In some embodiments, proximity ligation can include a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Patent No. 7,264,929).

A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation

As used herein, the term "splint" is an oligonucleotide that, when hybridized to other polynucleotides, acts as a "splint" to position the polynucleotides next to one another so that they can be ligated together. In some embodiments, the splint is DNA or RNA. The splint can include a nucleotide sequence that is partially complimentary to nucleotide sequences from two or more different oligonucleotides. In some embodiments, the splint assists in ligating a "donor" oligonucleotide and an "acceptor" oligonucleotide. In general, an RNA ligase, a DNA ligase, or another other variety of ligase is used to ligate two nucleotide sequences together.

In some embodiments, the splint is between 6 and 50 nucleotides in length, e.g., between 6 and 45, 6 and 40, 6 and 35, 6 and 30, 6 and 25, or 6 and 20 nucleotides in length. In some embodiments, the splint is between 10 and 50 nucleotides in length, e.g., between 10 and 45, 10 and 40, 10 and 35, 10 and 30, 10 and 25, or 10 and 20 nucleotides in length. In some embodiments, the splint is between 15 and 50, 15 and 45, 15 and 40, 15 and 35, 15 and 30, or 15 and 25 nucleotides in length.

A "feature" is an entity that acts as a support or repository for various molecular entities used in sample analysis. In some embodiments, some or all of the features in an array are functionalized for analyte capture. In some embodiments, functionalized features include one or more capture probe(s). Examples of features include, but are not limited to, a bead, a spot of any two- or three-dimensional geometry (e.g., an ink jet spot, a masked spot, a square on a grid), a well, and a hydrogel pad. In some embodiments, features are directly or indirectly attached or fixed to a substrate. In some embodiments, the features are not directly or indirectly attached or fixed to a substrate, but instead, for example, are disposed within an enclosed or partially enclosed three dimensional space (e.g., wells or divots).

The term "sequencing," as used herein, generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (e.g., single stranded DNA). Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). Alternatively or in addition, sequencing may be performed using nucleic acid amplification, polymerase chain reaction (PCR) (e.g., digital PCR, quantitative PCR, or real time PCR), or isothermal amplification. Such systems may provide a plurality of raw genetic data corresponding to the genetic information of a subject (e.g., human), as generated by the systems from a sample provided by the subject. In some examples, such systems provide sequencing reads (also "reads" herein). A read may include a string of nucleic acid bases corresponding to a sequence of a nucleic acid molecule that has been sequenced. In some situations, systems and methods provided herein may be used with proteomic information.

The term "template" as used herein generally refers to individual polynucleotide molecules from which another nucleic acid, including a complementary nucleic acid strand, can be synthesized by a nucleic acid polymerase. In addition, the template can be one or both strands of the polynucleotides that are capable of acting as a templates for template-dependent nucleic acid polymerization catalyzed by the nucleic acid polymerase. Use of this term should not be taken as limiting the scope of the present disclosure to polynucleotides which are actually used as a templates in a subsequent enzyme-catalyzed polymerization reaction. The template can be an RNA or DNA. The template can be cDNA corresponding to an RNA sequence. The template can be DNA.

As used herein, "amplification" of a template nucleic acid generally refers to a process of creating (e.g., in vitro) nucleic acid strands that are identical or complementary to at least a portion of a template nucleic acid sequence, or a universal or tag sequence that serves as a surrogate for the template nucleic acid sequence, all of which are only made if the template nucleic acid is present in a sample. Typically, nucleic acid amplification uses one or more nucleic acid polymerase and/or transcriptase enzymes to produce multiple copies of a template nucleic acid or fragments thereof, or of a sequence complementary to the template nucleic acid or fragments thereof. In vitro nucleic acid amplification techniques are may include transcription-associated amplification methods, such as Transcription-Mediated Amplification (TMA) or Nucleic Acid Sequence-Based Amplification (NASBA), and other methods such as Polymerase Chain Reaction (PCR), Reverse Transcriptase-PCR (RT-PCR), Replicase Mediated Amplification, and Ligase Chain Reaction (LCR).

The terms "polynucleotide," "polynucleotide," and "nucleic acid molecule", used interchangeably herein, refer to polymeric forms of nucleotides of any length, either ribonucleotides or deoxyribonucleotides. Thus, this term comprises, but is not limited to, single-, double-, or multi- stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups.

"Hybridization" as used herein may refer to the process in which two single-stranded polynucleotides bind non-covalently to form a stable double-stranded polynucleotide. In one aspect, the resulting double-stranded polynucleotide can be a "hybrid" or "duplex." "Hybridization conditions" typically include salt concentrations of approximately less than 1 M, often less than about 500 mM and may be less than about 200 mM. A "hybridization buffer" includes a buffered salt solution such as 5% SSPE, or other such buffers known in the art. Hybridization temperatures can be as low as 5°C, but are typically greater than 22°C, and more typically greater than about 30°C, and typically in excess of 37°C. Hybridizations are often performed under stringent conditions, *e.g.,* conditions under which a sequence will hybridize to its target sequence but will not hybridize to other, non-complementary sequences. Stringent conditions are sequence-dependent and are different in different circumstances. For example, longer fragments may require higher hybridization temperatures for specific hybridization than short fragments. As other factors may affect the stringency of hybridization, including base composition and length of the complementary strands, presence of organic solvents, and the extent of base mismatching, the combination of parameters is more important than the absolute measure of any one parameter alone. Generally stringent conditions are selected to be about 5°C lower than the T*ₘ* for the specific sequence at a defined ionic strength and pH. The melting temperature T*ₘ* can be the temperature at which a population of double-stranded nucleic acid molecules becomes half dissociated into single strands. Several equations for calculating the T*ₘ* of nucleic acids are well known in the art. As indicated by standard references, a simple estimate of the T*ₘ* value may be calculated by the equation, T*ₘ* =81.5 + 0.41 (% G + C), when a nucleic acid is in aqueous solution at 1 M NaCl (see *e.g.,* Anderson and Young, Quantitative Filter Hybridization, in Nucleic Acid Hybridization (1985)). Other references (*e.g.,* Allawi and SantaLucia, Jr., Biochemistry, 36:10581-94 (1997)) include alternative methods of computation which take structural and environmental, as well as sequence characteristics into account for the calculation of T*ₘ*.

In general, the stability of a hybrid is a function of the ion concentration and temperature. Typically, a hybridization reaction is performed under conditions of lower stringency, followed by washes of varying, but higher, stringency. Exemplary stringent conditions include a salt concentration of at least 0.01 M to no more than 1 M sodium ion concentration (or other salt) at a pH of about 7.0 to about 8.3 and a temperature of at least 25°C. For example, conditions of 5 × SSPE (750 mM NaCl, 50 mM sodium phosphate, 5 mM EDTA at pH 7.4) and a temperature of approximately 30°C are suitable for allele-specific hybridizations, though a suitable temperature depends on the length and/or GC content of the region hybridized. In one aspect, "stringency of hybridization" in determining percentage mismatch can be as follows: 1) high stringency: 0.1 × SSPE, 0.1% SDS, 65°C; 2) medium stringency: 0.2 × SSPE, 0.1% SDS, 50°C (also referred to as moderate stringency); and 3) low stringency: 1.0 × SSPE, 0.1% SDS, 50°C. It is understood that equivalent stringencies may be achieved using alternative buffers, salts and temperatures. For example, moderately stringent hybridization can refer to conditions that permit a nucleic acid molecule such as a probe to bind a complementary nucleic acid molecule. The hybridized nucleic acid molecules generally have at least 60% identity, including for example at least any of 70%, 75%, 80%, 85%, 90%, or 95% identity. Moderately stringent conditions can be conditions equivalent to hybridization in 50% formamide, 5 × Denhardt's solution, 5x SSPE, 0.2% SDS at 42°C, followed by washing in 0.2 × SSPE, 0.2% SDS, at 42°C. High stringency conditions can be provided, for example, by hybridization in 50% formamide, 5 × Denhardt's solution, 5 × SSPE, 0.2% SDS at 42°C, followed by washing in 0.1 × SSPE, and 0.1% SDS at 65°C. Low stringency hybridization can refer to conditions equivalent to hybridization in 10% formamide, 5 × Denhardt's solution, 6 × SSPE, 0.2% SDS at 22°C, followed by washing in 1x SSPE, 0.2% SDS, at 37°C. Denhardt's solution contains 1% Ficoll, 1% polyvinylpyrolidone, and 1% bovine serum albumin (BSA). 20 × SSPE (sodium chloride, sodium phosphate, ethylene diamide tetraacetic acid (EDTA)) contains 3M sodium chloride, 0.2M sodium phosphate, and 0.025 M EDTA. Other suitable moderate stringency and high stringency hybridization buffers and conditions are well known to those of skill in the art and are described, for example, in Sambrook et al., Molecular Cloning: A Laboratory Manual, 2nd ed., Cold Spring Harbor Press, Plainview, N.Y. (1989); and Ausubel et al., Short Protocols in Molecular Biology, 4th ed., John Wiley & Sons (1999).

Alternatively, substantial complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. *See* M. Kanehisa, Nucleic Acids Res. 12:203 (1984).

A "primer" used herein can be an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Primers usually are extended by a DNA polymerase.

"Ligation" may refer to the formation of a covalent bond or linkage between the termini of two or more nucleic acids, *e.g.,* oligonucleotides and/or polynucleotides, in a template-driven reaction. The nature of the bond or linkage may vary widely and the ligation may be carried out enzymatically or chemically. As used herein, ligations are usually carried out enzymatically to form a phosphodiester linkage between a 5' carbon terminal nucleotide of one oligonucleotide with a 3' carbon of another nucleotide.

"Sequencing," "sequence determination" and the like means determination of information relating to the nucleotide base sequence of a nucleic acid. Such information may include the identification or determination of partial as well as full sequence information of the nucleic acid. Sequence information may be determined with varying degrees of statistical reliability or confidence. In one aspect, the term includes the determination of the identity and ordering of a plurality of contiguous nucleotides in a nucleic acid. "High throughput digital sequencing" or "next generation sequencing" means sequence determination using methods that determine many (typically thousands to billions) of nucleic acid sequences in an intrinsically parallel manner, *e.g.* where DNA templates are prepared for sequencing not one at a time, but in a bulk process, and where many sequences are read out preferably in parallel, or alternatively using an ultra-high throughput serial process that itself may be parallelized. Such methods include but are not limited to pyrosequencing (for example, as commercialized by 454 Life Sciences, Inc., Branford, Conn.); sequencing by ligation (for example, as commercialized in the SOLiD^{™} technology, Life Technologies, Inc., Carlsbad, Calif.); sequencing by synthesis using modified nucleotides (such as commercialized in TruSeq^{™} and HiSeq^{™} technology by Illumina, Inc., San Diego, Calif.; HeliScope^{™} by Helicos Biosciences Corporation, Cambridge, Ma.; and PacBio RS by Pacific Biosciences of California, Inc., Menlo Park, Calif.), sequencing by ion detection technologies (such as Ion Torrent^{™} technology, Life Technologies, Carlsbad, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods.

"Multiplexing" or "multiplex assay" herein may refer to an assay or other analytical method in which the presence and/or amount of multiple targets, *e.g.,* multiple nucleic acid target sequences, can be assayed simultaneously by using more than one capture probe conjugate, each of which has at least one different detection characteristic, *e.g.,* fluorescence characteristic (for example excitation wavelength, emission wavelength, emission intensity, FWHM (full width at half maximum peak height), or fluorescence lifetime) or a unique nucleic acid or protein sequence characteristic.

The present disclosure is not intended to be limited in scope to the particular disclosed embodiments, which are provided, for example, to illustrate various aspects of the present disclosure. Various modifications to the compositions and methods described will become apparent from the description and teachings herein.

## Claims

1. A method for generating an immobilized nucleic acid, comprising:
a) delivering a first oligonucleotide of at least four nucleotides in length through a first microfluidic channel to a corresponding first area on a substrate, whereby the first oligonucleotide is attached to an oligonucleotide molecule immobilized in the first area to generate an extended oligonucleotide molecule, optionally wherein the first oligonucleotide comprises a first barcode sequence;
b) delivering a second oligonucleotide of at least four nucleotides in length through a second microfluidic channel to a corresponding second area on the substrate, whereby the second oligonucleotide is attached to the extended oligonucleotide molecule to generate a further extended oligonucleotide molecule in an overlapping area between the first and second areas, optionally wherein the second oligonucleotide comprises a second barcode sequence;
c) irradiating the substrate to render oligonucleotide molecules in one or more regions on the substrate available for oligonucleotide attachment, whereas oligonucleotide molecules in one or more other regions on the substrate are not available for oligonucleotide attachment; and
d) attaching a third oligonucleotide of at least four nucleotides in length to the further extended oligonucleotide molecule to generate an immobilized nucleic acid on the substrate, optionally wherein the third oligonucleotide comprises a third barcode sequence and the immobilized nucleic acid comprises the first, second, and third barcode sequences.

2. The method of claim 1, wherein the first oligonucleotide comprises a sequence that hybridizes to a first splint which in turn hybridizes to the oligonucleotide molecule, and wherein the first oligonucleotide is ligated to the oligonucleotide molecule using the first splint as a template to generate the extended oligonucleotide molecule.

3. The method of claim 2, wherein the second oligonucleotide comprises a sequence that hybridizes to a second splint which in turn hybridizes to the extended oligonucleotide molecule, and wherein the second oligonucleotide is ligated to the extended oligonucleotide molecule using the second splint as a template to generate the further extended oligonucleotide molecule.

4. The method of any one of claims 1-3, wherein prior to the irradiating in c), the oligonucleotide molecules in the one or more regions are protected from hybridization and/or ligation.

5. The method of any one of claims 1-4, wherein during and after the irradiating in c), the oligonucleotide molecules in the one or more other regions are protected from hybridization and/or ligation.

6. The method of claim 4 or 5, wherein:
(i) the oligonucleotide molecules are protected from hybridization and/or ligation by a photoresist covering the oligonucleotide molecules, optionally wherein the photoresist in irradiated regions is removed and the photoresist in masked or non-irradiated regions is not removed;
(ii) the oligonucleotide molecules are protected from hybridization and/or ligation by a photo-cleavable protective group of each oligonucleotide molecule, optionally wherein the photo-cleavable protective group in the irradiated regions is cleaved and the photo-cleavable protective group in masked or non-irradiated regions is not cleaved; or
(iii) the oligonucleotide molecules are protected from hybridization and/or ligation by a photo-cleavable polymer binding to the oligonucleotide molecules, optionally wherein the photo-cleavable polymer in the irradiated regions is cleaved and the photo-cleavable polymer in masked or non-irradiated regions is not cleaved.

7. The method of any one of claims 1-6, wherein the substrate is irradiated through a photomask comprising openings that correspond to regions on the substrate, and one or more of the regions are in the overlapping area between the first area and the second area.

8. The method of any one of claims 1-7, comprising irradiating the substrate in multiple cycles, each cycle for irradiating one or more regions that are different from the region(s) irradiated in another cycle.

9. The method of claim 8, comprising irradiating the overlapping area between the first area and the second area in multiple cycles, each cycle for irradiating one or more regions in the overlapping area that are different from the region(s) irradiated in another cycle.

10. The method of any one of claims 7-9, comprising translating the photomask from a first position to a second position relative to the substrate, each position for a cycle of irradiating the substrate.

11. The method of any one of claims 1-10, wherein the first and second microfluidic channels form an angle of about 90 degrees, about 80 degrees, about 70 degrees, about 60 degrees, about 50 degrees, about 40 degrees, about 30 degrees, about 20 degrees, or about 10 degrees.

12. The method of any one of claims 1-11, wherein the first and second microfluidic channels are provided in the same microfluidic device or in separate microfluidic devices.

13. The method of any one of claims 1-12, wherein the width of the first and/or second microfluidic channels is about 5 µm, about 10 µm, about 50 µm, about 100 µm, about 150 µm, about 200 µm, about 250 µm, about 300 µm, about 350 µm, about 400 µm, about 450 µm, or about 500 µm.

14. The method of any one of claims 1-13, wherein the depth of the first and/or second microfluidic channels is about 5 µm, about 10 µm, about 50 µm, about 100 µm, about 150 µm, about 200 µm, about 250 µm, about 300 µm, about 350 µm, about 400 µm, about 450 µm, or about 500 µm.

15. The method of any one of claims 1-14, wherein the substrate is a chip, a wafer, a die, or a slide and the immobilized nucleic acid is generated in the absence of a cell or tissue sample on the substrate.

## Patentansprüche

1. Verfahren zum Erzeugen einer immobilisierten Nukleinsäure, umfassend:
a)Zuführen eines ersten Oligonukleotids von mindestens vier Nukleotiden in der Länge durch einen ersten mikrofluidischen Kanal zu einem entsprechenden ersten Bereich auf einem Substrat, wobei das erste Oligonukleotid an ein Oligonukleotid-Molekül angelagert ist, das in dem ersten Bereich immobilisiert ist, um ein erweitertes Oligonukleotid-Molekül zu erzeugen, wobei das erste Oligonukleotid optional eine erste Barcode-Sequenz umfasst;
b)Zuführen eines zweiten Oligonukleotids mit einer Länge von mindestens vier Nukleotiden durch einen zweiten mikrofluidischen Kanal zu einem entsprechenden zweiten Bereich auf dem Substrat, wobei das zweite Oligonukleotid an das verlängerte Oligonukleotid-Molekül angelagert ist, um ein weiteres verlängertes Oligonukleotid-Molekül in einem überlappenden Bereich zwischen dem ersten und dem zweiten Bereich zu erzeugen, wobei das zweite Oligonukleotid optional eine zweite Barcode-Sequenz umfasst;
c)Bestrahlen des Substrats, um Oligonukleotid-Moleküle in einer oder mehreren Regionen auf dem Substrat für die Anlagerung von Oligonukleotiden verfügbar zu machen, während Oligonukleotid-Moleküle in einer oder mehreren anderen Regionen auf dem Substrat nicht für die Anlagerung von Oligonukleotiden verfügbar sind; und
d)Anlagern eines dritten Oligonukleotids mit einer Länge von mindestens vier Nukleotiden an das weitere verlängerte Oligonukleotid-Molekül, um eine immobilisierte Nukleinsäure auf dem Substrat zu erzeugen, wobei das dritte Oligonukleotid optional eine dritte Barcode-Sequenz umfasst und die immobilisierte Nukleinsäure die erste, zweite und dritte Barcode-Sequenzen umfasst.

2. Verfahren nach Anspruch 1, wobei das erste Oligonukleotid eine Sequenz umfasst, die an einen ersten Splint hybridisiert, der wiederum an das Oligonukleotid-Molekül hybridisiert, und wobei das erste Oligonukleotid an das Oligonukleotid-Molekül unter Verwendung des ersten Splints als Matrize zur Erzeugung des verlängerten Oligonukleotid-Moleküls ligiert wird.

3. Verfahren nach Anspruch 2, wobei das zweite Oligonukleotid eine Sequenz umfasst, die an einen zweiten Splint hybridisiert, der wiederum an das verlängerte Oligonukleotid-Molekül hybridisiert, und wobei das zweite Oligonukleotid an das verlängerte Oligonukleotid-Molekül unter Verwendung des zweiten Splints als Matrize zur Erzeugung des weiteren verlängerten Oligonukleotid-Moleküls ligiert wird.

4. Verfahren nach einem der Ansprüche 1 bis 3, wobei vor dem Bestrahlen in c) die Oligonukleotid-Moleküle in der einen oder den mehreren der Regionen gegen Hybridisierung bzw. Ligation geschützt werden.

5. Verfahren nach einem der Ansprüche 1 bis 4, wobei während und nach der Bestrahlung in c) die Oligonukleotid-Moleküle in der einen oder den mehreren anderen Regionen gegen Hybridisierung bzw. Ligation geschützt werden.

6. Verfahren nach Anspruch 4 oder 5, wobei:
(i) die Oligonukleotid-Moleküle durch einen Fotolack, der die Oligonukleotid-Moleküle bedeckt, vor Hybridisierung bzw. Ligation geschützt sind, wobei optional der Fotolack in bestrahlten Bereichen entfernt wird und der Fotolack in maskierten oder nicht bestrahlten Bereichen nicht entfernt wird;
(ii) die Oligonukleotid-Moleküle durch eine fotospaltbare Schutzgruppe jedes Oligonukleotid-Moleküls vor Hybridisierung bzw. Ligation geschützt sind, wobei optional die fotospaltbare Schutzgruppe in den bestrahlten Bereichen gespalten wird und die fotospaltbare Schutzgruppe in maskierten oder nicht bestrahlten Bereichen nicht gespalten wird; oder
(iii) die Oligonukleotid-Moleküle durch ein fotospaltbares Polymer, das sich an die Oligonukleotid-Moleküle bindet, vor Hybridisierung bzw. Ligation geschützt sind, wobei gegebenenfalls das fotospaltbare Polymer in den bestrahlten Bereichen gespalten wird und das fotospaltbare Polymer in maskierten oder nicht bestrahlten Bereichen nicht gespalten wird.

7. Verfahren nach einem der Ansprüche 1 bis 6, wobei das Substrat durch eine Fotomaske bestrahlt wird, die Öffnungen umfasst, die Regionen auf dem Substrat entsprechen und sich eine oder mehrere der Regionen im überlappenden Bereich zwischen dem ersten und dem zweiten Bereich befinden.

8. Verfahren nach einem der Ansprüche 1 bis 7, umfassend das Bestrahlen des Substrats in mehreren Zyklen, wobei jeder Zyklus zum Bestrahlen eines oder mehrerer Bereiche, die sich von dem/den in einem anderen Zyklus bestrahlten Bereich(en) unterscheiden, vorgesehen ist.

9. Verfahren nach Anspruch 8, umfassend die Bestrahlung des Überlappungsbereichs zwischen dem ersten Bereich und dem zweiten Bereich in mehreren Zyklen, wobei jeder Zyklus zum Bestrahlen eines oder mehrerer Bereiche in dem Überlappungsbereich, die sich von dem/den in einem anderen Zyklus bestrahlten Bereich(en) unterscheiden, vorgesehen ist.

10. Verfahren nach einem der Ansprüche 7 bis 9, umfassend das Verschieben der Fotomaske aus einer ersten Position in eine zweite Position relativ zum Substrat, wobei jede Position einem Bestrahlungszyklus des Substrats entspricht.

11. Verfahren nach einem der Ansprüche 1 bis 10, wobei der erste und der zweite mikrofluidische Kanal einen Winkel von etwa 90 Grad, etwa 80 Grad, etwa 70 Grad, etwa 60 Grad, etwa 50 Grad, etwa 40 Grad, etwa 30 Grad, etwa 20 Grad oder etwa 10 Grad bilden.

12. Verfahren nach einem der Ansprüche 1-11, wobei der erste und der zweite mikrofluidische Kanal in derselben mikrofluidischen Vorrichtung oder in getrennten mikrofluidischen Vorrichtungen vorgesehen sind.

13. Verfahren nach einem der Ansprüche 1 bis 12, wobei die Breite des ersten und/oder zweiten mikrofluidischen Kanals etwa 5 pm, etwa 10 pm, etwa 50 pm, etwa 100 pm, etwa 150 pm, etwa 200 pm, etwa 250 pm, etwa 300 pm, etwa 350 pm, etwa 400 pm, etwa 450 pm oder etwa 500 pm beträgt.

14. Verfahren nach einem der Ansprüche 1 bis 13, wobei die Tiefe des ersten und/oder zweiten mikrofluidischen Kanals etwa 5 pm, etwa 10 pm, etwa 50 pm, etwa 100 pm, etwa 150 pm, etwa 200 pm, etwa 250 pm, etwa 300 pm, etwa 350 pm, etwa 400 pm, etwa 450 pm oder etwa 500 pm beträgt.

15. Verfahren nach einem der Ansprüche 1-14, wobei das Substrat ein Chip, ein Wafer, ein Formstück oder ein Objektträger ist und die immobilisierte Nukleinsäure in Abwesenheit einer Zell- oder Gewebeprobe auf dem Substrat erzeugt wird.

## Revendications

1. Procédé pour générer un acide nucléique immobilisé, comprenant :
a)la délivrance d'un premier oligonucléotide de l'au moins quatre nucléotides de longueur par l'intermédiaire d'un premier canal microfluidique vers une première zone correspondante sur un substrat, moyennant quoi le premier oligonucléotide est fixé à une molécule d'oligonucléotide immobilisée dans la première zone pour générer une molécule d'oligonucléotide étendue, facultativement dans lequel le premier oligonucléotide comprend une première séquence de code à barres ;
b)la délivrance d'un deuxième oligonucléotide de l'au moins quatre nucléotides de longueur par l'intermédiaire d'un deuxième canal microfluidique vers une deuxième zone correspondante sur le substrat, moyennant quoi le deuxième oligonucléotide est fixé à la molécule d'oligonucléotide étendue pour générer une molécule d'oligonucléotide étendue supplémentaire dans une zone de chevauchement entre les première et deuxième zones, dans lequel facultativement le deuxième oligonucléotide comprend une deuxième séquence de code à barres ;
c)l'irradiation du substrat pour rendre des molécules d'oligonucléotides dans une ou plusieurs régions sur le substrat disponible pour la fixation d'oligonucléotides, tandis que des molécules d'oligonucléotides dans une ou plusieurs autres régions sur le substrat ne sont pas disponibles pour la fixation d'oligonucléotides ; et
d)la fixation d'un troisième oligonucléotide de l'au moins quatre nucléotides de longueur à la molécule d'oligonucléotide étendue supplémentaire pour générer un acide nucléique immobilisé sur le substrat, dans lequel facultativement le troisième oligonucléotide comprend une troisième séquence de code à barres et l'acide nucléique immobilisé comprend les première, deuxième et troisième séquences de code à barres.

2. Procédé selon la revendication 1, dans lequel le premier oligonucléotide comprend une séquence qui s'hybride à une première attelle qui à son tour s'hybride à la molécule d'oligonucléotide, et dans lequel le premier oligonucléotide est ligaturé à la molécule d'oligonucléotide en utilisant la première attelle comme matrice pour générer la molécule d'oligonucléotide étendue.

3. Procédé selon la revendication 2, dans lequel le deuxième oligonucléotide comprend une séquence qui s'hybride à une deuxième attelle qui s'hybride à son tour à la molécule d'oligonucléotide étendue, et dans lequel le deuxième oligonucléotide est ligaturé à la molécule d'oligonucléotide étendue en utilisant la deuxième attelle comme matrice pour générer la molécule d'oligonucléotide étendue supplémentaire.

4. Procédé selon l'une quelconque des revendications 1 à 3, dans lequel, avant l'irradiation à l'étape c), les molécules d'oligonucléotides dans les une ou plusieurs régions sont protégées de l'hybridation et/ou de la ligature.

5. Procédé selon l'une quelconque des revendications 1 à 4, dans lequel, pendant et après l'irradiation à l'étape c), les molécules d'oligonucléotides dans les une ou plusieurs autres régions sont protégées de l'hybridation et/ou de la ligature.

6. Procédé selon la revendication 4 ou 5, dans lequel :
(i) les molécules d'oligonucléotides sont protégées de l'hybridation et/ou de la ligature par une résine photosensible recouvrant les molécules d'oligonucléotides, dans lequel facultativement la résine photosensible dans les régions irradiées est éliminée et la résine photosensible dans les régions masquées ou non irradiées n'est pas éliminée ;
(ii) les molécules d'oligonucléotides sont protégées de l'hybridation et/ou de la ligature par un groupe protecteur photoclivable de chaque molécule d'oligonucléotide, dans lequel facultativement le groupe protecteur photoclivable dans les régions irradiées est clivé et le groupe protecteur photoclivable dans les régions masquées ou non irradiées n'est pas clivé ; ou
(iii) les molécules d'oligonucléotides sont protégées de l'hybridation et/ou de la ligature par un polymère photoclivable se liant aux molécules d'oligonucléotides, facultativement dans lequel le polymère photoclivable dans les régions irradiées est clivé et le polymère photoclivable dans les régions masquées ou non irradiées n'est pas clivé.

7. Procédé selon l'une quelconque des revendications 1 à 6, dans lequel le substrat est irradié à travers un photomasque comprenant des ouvertures qui correspondent à des régions sur le substrat, et une ou plusieurs des régions sont dans la zone de chevauchement entre la première zone et la deuxième zone.

8. Procédé selon l'une quelconque des revendications 1 à 7, comprenant l'irradiation du substrat en cycles multiples, chaque cycle étant destiné à irradier une ou plusieurs régions qui sont différentes de la ou des régions irradiées dans un autre cycle.

9. Procédé selon la revendication 8, comprenant l'irradiation de la zone de chevauchement entre la première zone et la deuxième zone en cycles multiples, chaque cycle étant destiné à irradier une ou plusieurs régions dans la zone de chevauchement qui sont différentes de la ou des régions irradiées dans un autre cycle.

10. Procédé selon l'une quelconque des revendications 7 à 9, comprenant la translation du photomasque d'une première position à une deuxième position par rapport au substrat, chaque position étant pour un cycle d'irradiation du substrat.

11. Procédé selon l'une quelconque des revendications 1 à 10, dans lequel les premier et deuxième canaux microfluidiques forment un angle d'environ 90 degrés, environ 80 degrés, environ 70 degrés, environ 60 degrés, environ 50 degrés, environ 40 degrés, environ 30 degrés, environ 20 degrés ou environ 10 degrés.

12. Procédé selon l'une quelconque des revendications 1 à 11, dans lequel les premier et deuxième canaux microfluidiques sont prévus dans le même dispositif microfluidique ou dans des dispositifs microfluidiques distincts.

13. Procédé selon l'une quelconque des revendications 1 à 12, dans lequel la largeur des premier et/ou deuxième canaux microfluidiques est d'environ 5 µm, environ 10 µm, environ 50 µm, environ 100 µm, environ 150 µm, environ 200 µm, environ 250 µm, environ 300 µm, environ 350 µm, environ 400 µm, environ 450 µm, ou environ 500 µm.

14. Procédé selon l'une quelconque des revendications 1 à 13, dans lequel la profondeur des premier et/ou deuxième canaux microfluidiques est d'environ 5 µm, environ 10 µm, environ 50 µm, environ 100 µm, environ 150 µm, environ 200 µm, environ 250 µm, environ 300 µm, environ 350 µm, environ 400 µm, environ 450 µm, ou environ 500 µm.

15. Procédé selon l'une quelconque des revendications 1 à 14, dans lequel le substrat est une puce, une plaquette, une matrice ou une lame et l'acide nucléique immobilisé est généré en l'absence d'un échantillon de cellule ou de tissu sur le substrat.
